(19) Europäisches Patentamt / European Patent Office / Office européen des brevets

(11) EP 2 751 065 B1

(12) EUROPÄISCHE PATENTSCHRIFT

(45) Veröffentlichungstag und Bekanntmachung des Hinweises auf die Patenterteilung:
**16.11.2016 Patentblatt 2016/46**

(21) Anmeldenummer: **12750400.9**

(22) Anmeldetag: **27.08.2012**

(51) Int Cl.:
***C07C 209/48*** (2006.01) ***C07C 211/14*** (2006.01)

(86) Internationale Anmeldenummer:
**PCT/EP2012/066619**

(87) Internationale Veröffentlichungsnummer:
**WO 2013/030161 (07.03.2013 Gazette 2013/10)**

# (54) VERFAHREN ZUR HERSTELLUNG VON EDDN UND/ODER EDMN DURCH UMSETZUNG VON EDFA UND/ODER EDMFA MIT HCN

METHOD FOR PRODUCING EDDN AND/OR EDMN THROUGH THE CONVERSION OF EDFA AND/OR EDMFA WITH HCN

PROCÉDÉ DE FABRICATION D'EDDN ET/OU EDMN PAR CONVERSION D'EDFA ET/OU EDMFA AVEC HCN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.08.2011 EP 11179597**

(43) Veröffentlichungstag der Anmeldung:
**09.07.2014 Patentblatt 2014/28**

(73) Patentinhaber: **BASF SE**
**67056 Ludwigshafen am Rhein (DE)**

(72) Erfinder:
- **LUYKEN, Hermann**
  **67069 Ludwigshafen (DE)**
- **AHRENS, Sebastian**
  **69168 Wiesloch (DE)**
- **BRASCHE, Gordon**
  **60594 Frankfurt (DE)**
- **BALDAMUS, Jens**
  **67067 Ludwigshafen (DE)**
- **BAUMANN, Robert**
  **68529 Mannheim (DE)**
- **HUGO, Randolf**
  **200127 Shanghai Pu Dong New District (CN)**
- **JAEGLI, Stephanie**
  **68163 Mannheim (DE)**
- **MELDER, Johann-Peter**
  **67459 Böhl-Iggelheim (DE)**
- **PASTRE, Jörg**
  **64625 Bensheim (DE)**
- **BUSCHHAUS, Boris**
  **69117 Heidelberg (DE)**

(74) Vertreter: **BASF IP Association**
**BASF SE**
**ZRX-C6**
**67056 Ludwigshafen (DE)**

(56) Entgegenhaltungen:
**WO-A2-2008/104582 US-A- 3 947 522**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 751 065 B1

## Beschreibung

Die vorliegende Anmeldung schließt durch Verweis die am 31.08.2011 eingereichte vorläufige US-Anmeldung 61/529290 ein.

Die vorliegende Erfindung betrifft ein Verfahren zur Herstellung von EDDN und/oder EDMN durch Umsetzung von HCN und EDFA und/oder EDMFA, wobei die Umsetzung in in einem Reaktor mit begrenzter Rückvermischung und kurzer Verweilzeit erfolgt. Die vorliegende Erfindung betrifft zudem die Herstellung von TETA und/oder DETA durch Umsetzung des so hergestellten EDDN bzw. EDMN mit Wasserstoff in Gegenwart eines Katalysators, dadurch gekennzeichnet, dass nach der Herstellung von EDDN bzw. EDMN eine Abreicherung von Wasser durch Destillation durchgeführt wird. Ein weiterer Gegenstand der vorliegenden Erfindung ist die Herstellung von Epoxydharzen, Amiden oder Polyamiden aus dem erfindungsgemäß erhaltenen DETA bzw. TETA.

In der WO 2008/104579 und in dem in der WO 2008/104579 genannten Stand der Technik werden verschiedene Herstellungsmethoden für EDDN bzw. EDMN genannt.
In der WO 2008/104579 wird EDDN durch Umsetzung von EDA mit Formaldehyd (FA) und Blausäure (HCN) hergestellt, wobei das molare Verhältnis von EDA zu FA zu HCN 1 : 1,5 : 1,5 bis 1 : 2 : 2 [mol : mol : mol] beträgt.
Die Herstellung kann dadurch erfolgen, dass a) EDA mit FACH umgesetzt wird, wobei das molare Verhältnis von EDA zu FACH 1 : 1,5 bis 1 : 2 beträgt, oder dass b) EDDN durch Umsetzung eines Ethylendiamin-Formaldehyd-Addukts (EDFA) mit Blausäure hergestellt wird, wobei das molare Verhältnis von EDFA zu HCN 1 : 1,5 bis 1 : 2 beträgt, oder dass c) EDA mit einem Gemisch aus Formaldehyd und Blausäure umgesetzt wird, wobei das molare Verhältnis von EDA zu FA zu HCN 1 : 1,5 : 1,5 bis 1 : 2 : 2 beträgt, oder dass d) EDA zeitgleich mit Formaldehyd und HCN umgesetzt wird, wobei das molare Verhältnis von EDA zu FA zu HCN 1 : 1,5 : 1,5 bis 1 : 2 : 2 beträgt.
Es wird offenbart, dass diese Umsetzungen vorzugsweise bei einer Temperatur von 10 bis 90°C und bei Normaldruck bis leicht erhöhtem Überdruck durchgeführt werden Als bevorzugte Reaktoren werden ein Rohrreaktor oder eine Rührkesselkaskade beschrieben. Die Aufarbeitung des entstandenen Reaktionsaustrags erfolgt bevorzugt destillativ, wobei in einer ersten Stufe zunächst Leichtsieder, wie Blausäure, abgetrennt werden und in einem zweiten Destillationsschritt Wasser entfernt wird. Das verbleibende Aminonitrilgemisch kann noch einen Restwassergehalt von bevorzugt mindestens 10 Gew.-% aufweisen.

Im Rahmen der vorliegenden Erfindung wurde gefunden, dass insbesondere wässrige Lösungen von EDDN bzw. EDMN thermisch instabil sind. EDDN und EDMN können Zersetzungsprodukte bilden, die die Ausbeute an EDDN bzw. EDMN verringern können und in Folgereaktion, beispielsweise der nachfolgenden Hydrierung zu TETA und/oder DETA zu einer verschlechterten Verarbeitungsqualität, insbesondere zu einer Erhöhung der Verfärbung, führen können.

Die Aufgabe der vorliegenden Erfindung bestand darin, ein Verfahren zur Herstellung von EDDN und/oder EDMN zur Verfügung zu stellen, das die Herstellung von EDDN und/oder EDMN mit hohen Ausbeuten, Umsätzen und Selektivitäten im großtechnischen Maßstab ermöglicht. Der Anteil an unerwünschten Nebenprodukten, insbesondere an Zersetzungsprodukten von EDDN und/oder EDMN, sollte gegenüber dem Stand der Technik verringert werden. Weiterhin sollte EDDN bzw. EDMN zur Verfügung gestellt werden, das in Folgereaktionen, wie der Hydrierung zu TETA bzw. DETA, im geringeren Maße zu Produktverfärbungen führt und eine längere Standzeit des Hydrierkatalysators ermöglicht.

Die Aufgabe wurde gelöst durch ein
Verfahren zur Umsetzung von Ethylendiamin-Formaldehyd-Addukt (EDFA) und/oder Ethylendiamin-Monoformaldehyd-Addukt (EDMFA) mit Blausäure (HCN) in einem Reaktor mit begrenzter Rückvermischung bei einer Temperatur im Bereich von 20 bis 120°C, dadurch gekennzeichnet, dass die Verweilzeit im Reaktor 300 Sekunden oder weniger beträgt. EDDN und/oder EDMN wird durch Umsetzung von FA, HCN und EDA hergestellt, wobei zunächst FA mit EDA zu EDFA und/oder EDMFA umgesetzt wird, welches dann mit HCN weiter zu EDDN und/oder EDMN reagieren kann.

EDA

EDA kann nach dem EDC (Ethylendichlorid)-Verfahren durch Umsetzung von Ethylendichlorid (EDC) mit Ammoniak in wässriger Phase hergestellt werden. Einzelheiten zu dem Verfahren sind beispielsweise im Ullmann aufgeführt (Artikel "Amines, aliphatic" in Ullmann's Encyclopedia of Industrial Chemistry, Karsten Eller, Erhard Henkes, Roland Rossbacher and Hartmut Höke, Published Online : 15 JUN 2000, DOI: 10.1002/14356007.a02_001, Seite 33).

Eine weitere Möglichkeit zur Herstellung von EDA besteht in der katalytischen Umsetzung von Monoethanolamin (MEOA) mit Ammoniak (Artikel "Amines, aliphatic" in Ullmann's Encyclopedia of Industrial Chemistry, Karsten Eller, Erhard Henkes, Roland Rossbacher and Hartmut Höke, Published Online : 15 JUN 2000, DOI: 10.1002/14356007.a02 001, Seite 33 oder Hans-Jürgen Arpe, Industrielle Organische Chemie, 6. Auflage (2007), Wiley VCH, 2007).

EDA kann auch durch Hydrierung von Aminoacetonitril (AAN) erhalten werden, wobei AAN durch Umsetzung von Blausäure, Formaldehyd (FA) und Ammoniak hergestellt werden kann. Die Hydrierung on AAN zu EDA ist beispielsweise in der WO 2008/104583 beschrieben.

Vorzugsweise wird EDA in Form seiner freien Base eingesetzt, gegebenenfalls können jedoch auch Salze wie das Dihydrochlorid von EDA, als Edukt verwendet werden.

Die Reinheit des in das Verfahren eingesetzten EDA beträgt vorzugsweise 95 Gew.-% und mehr, besonders bevorzugt 98 Gew.-% und mehr, ganz besonders bevorzugt 99 Gew.-% und mehr und insbesondere bevorzugt 99,5 Gew.-% und mehr.

FA

Als weiteres Edukt wird Formaldehyd eingesetzt.

Formaldehyd ist eine im Handel allgemein erhältliche Chemikalie.

Bevorzugt wird Formaldehyd als 30 bis 50%-ige wässrige Lösung eingesetzt.

HCN

Weiterhin wird Blausäure zur Herstellung von EDDN und/oder EDMN eingesetzt.

Blausäure ist ebenfalls eine im Handel allgemein erhältliche Chemikalie.

Blausäure kann großtechnisch nach im Wesentlichen drei verschiedenen Verfahren hergestellt werden. Nach einem ersten Verfahren kann Blausäure durch Ammoxidation von Methan mit Sauerstoff und Ammoniak (Andrussow-Verfahren) erhalten werden. Nach einem zweiten Verfahren kann Blausäure aus Methan und Ammoniak durch Ammondehydrierung in Abwesenheit von Sauerstoff erhalten werden. Schließlich kann Blausäure großtechnisch durch Dehydratisierung von Formamid hergestellt werden.

Der nach diesen Verfahren hergestellten Blausäure wird in der Regel ein saurer Stabilisator, beispielsweise $SO_2$, Schwefelsäure, Phosphorsäure oder eine organische Säure, wie Essigsäure, zugesetzt, um die autokatalytische Polymerisation von Blausäure, die in Rohrleitungen zu Verstopfungen führen kann, zu verhindern.

Blausäure kann flüssig oder gasförmig, in reiner Form oder als wässrige Lösung eingesetzt werden.

Bevorzugt wird Blausäure als 50 bis 95 Gew.-%ige, besonders bevorzugt als 75 bis 90 Gew.-%ige wässrige Lösung eingesetzt.

Blausäure wird vorzugsweise in einer Reinheit von mehr 90 Gew.-% oder mehr eingesetzt. Bevorzugt wird stabilisatorfreie HCN verwendet.

Wenn eine stabilisierte HCN eingesetzt wird, so ist es bevorzugt, dass der Stabilisator eine organische Säure, insbesondere Essigsäure, ist.

In einer bevorzugten Ausführungsform wird die EDDN-Herstellung im Wesentlichen frei von Cyanosalzen, wie KCN, durchgeführt.

Wasser

Die Umsetzung von EDA, HCN und FA findet bevorzugt in Gegenwart von Wasser statt.

Bei der Umsetzung von EDA, HCN und FA entsteht im Allgemeinen pro Mol eingesetztem Formaldehyd 1 Mol Wasser. Wasser kann aber auch zusätzlich zugeführt werden, beispielsweise in dem man die Edukte in Form ihrer wässrigen Lösungen einsetzt. Insbesondere wird, wie zuvor beschrieben im Allgemeinen FA und/oder HCN als wässrige Lösung zur Herstellung von EDDN bzw. EDMN eingesetzt.

Die Wassermenge liegt im Allgemeinen im Bereich von 1 bis 50 Mol pro Mol, bevorzugt im Bereich von 2 bis 40 Mol, und besonders bevorzugt im Bereich von 3 bis 30 Mol pro Mol eingesetztem EDA.

Erfolgt die Umsetzung von HCN, EDA und FA in einem adiabaten Reaktor, d.h. einen Reaktor, der im Wesentlichen nicht gekühlt wird und die Reaktionstemperatur durch die freiwerdende

Reaktionswärme erhöht wird, so ist es bevorzugt, dass EDA vor dem Einleiten in den adiabaten Reaktor und vor der Vermischung mit den anderen Ausgangsstoffen, wie FACH bzw. HCN oder FA, mit Wasser vermischt wird, da beim Mischen von EDA und Wasser in der Regel die Temperatur des wässrigen EDA-Stroms durch die Exothermie der sich bildenden Hydrate ansteigt. Durch Ableiten der EDA-Hydratationswärme vor dem Eintritt des EDA in den Reaktor, kann der Temperaturanstieg in dem adiabaten Reaktor verringert werden.

Als Vorrichtungen für die Mischung von EDA und Wasser sind statische Mischer, leere Leitungen mit turbulenter Strömung, Pumpen oder Wärmetauscher geeignet.

Zur Ableitung der Hydratationswärme wird Wasser mit EDA bevorzugt in einem molaren Verhältnis von Wasser zu EDA von 1 : 1 bis 6 : 1 vermischt.

Erfolgt die Umsetzung von HCN, EDA und FA in einem Reaktor, bei dem die entstehenden Reaktionswärme abgeführt werden kann, beispielsweise in einem Schlaufenreaktor mit externem Wärmetauscher, so ist es bevorzugt, dass neben dem Wasser, das üblicherweise mit den Einsatz von wässrigen Lösungen von FA und HCN in das Verfahren

eingebracht wird, kein zusätzliches Wasser zugeführt wird.

Organisches Lösungsmittel

Die Umsetzung von EDFA und/oder EDMFA und HCN findet bevorzugt in Gegenwart eines organischen Lösungsmittels statt.
Als organische Lösungsmittel werden bevorzugt solche, ausgewählt aus der Gruppe bestehend aus aliphatischen, cycloaliphatischen, araliphatischen, aromatischen Kohlenwasserstoffen, Alkoholen und Ethern verwendet.
Es ist besonders bevorzugt, dass das organische Lösungsmittel unter den Bedingungen einer nachfolgenden Hydrierung von EDDN und/oder EDMN beständig ist.
Außerdem ist es bevorzugt, dass das organische Lösungsmittel bei einem Druck im Bereich von 50 bis 500 mbar im Bereich von 20 bis 50°C kondensierbar ist, um normales Kühlwasser bei der nachfolgenden Aufarbeitung von EDDN bzw. EDMN zum Kondensieren verwenden zu können.
Weiterhin ist es bevorzugt, dass das organische Lösungsmittel niedrig genug siedet, um eine Sumpftemperatur von weniger als 100°C bei der nachfolgenden Wasserabtrennung während der Aufarbeitung des Reaktionsaustrags einstellen zu können.
Bevorzugte organische Lösungsmittel sind beispielsweise Cyclohexan, Methylcyclohexan, Toluol, N-Methylmorpholin, o-Xylol, m-Xylol oder p-Xylol, Anisol, n-Pentan, n-Hexan, n-Heptan, n-Octan, n-Nonan, Diisobutylether, Leichtbenzin, Benzin, Benzol, Diglyme, Tetrahydrofuran, 2- und 3- Methyltetrahydrofuran (MeTHF) und Cyclohexanol, oder Gemische dieser Verbindungen. Besonders bevorzugte Lösungsmittel sind Cyclohexan, Methylcyclohexan, Toluol, N-Methylmorpholin, o-Xylol, m-Xylol oder p-Xylol, Anisol, n-Pentan, n-Hexan, n-Heptan, n-Octan, n-Nonan, Diisobutylether, Leichtbenzin, Benzin (Benzol), Diglyme und MeTHF, oder Gemische dieser Verbindungen.
Die Menge an organischem Lösungsmittel beträgt im Allgemeinen 0,1 bis 50 kg pro kg, bevorzugt 1 bis 30 kg, besonders bevorzugt 3 bis 25 kg pro kg eingesetztem EDA.

In einer besonders bevorzugten Verfahrensvariante setzt man bei der Umsetzung von von ED-FA und/oder EDMFA und HCN ein organisches Lösungsmittel ein, welches einen Siedepunkt aufweist, der zwischen Wasser und EDDN bzw. EDMN liegt, insbesondere unter den Bedingungen der nachfolgend beschriebenen destillativen Wasserabreicherung. Wie nachfolgend beschrieben, ermöglichen organische Lösungsmittel, die in diesem Bereich sieden, eine besonders effiziente Abtrennung von Wasser aus dem Reaktionsaustrag, der bei der Umsetzung von von EDFA und/oder EDMFA und HCN erhalten wird. Besonders bevorzugte Lösungsmittel mit einem Siedepunkt, der zwischen Wasser und EDDN oder EDMN liegt, sind Toluol, N-Methylmorpholin, o-Xylol, m-Xylol oder p-Xylol, Anisol, n-Octan, n-Nonan, Diisobutylether und Diglyme, oder Mischungen davon.
Einige der voranstehend genannten organischen Lösungsmittel können mit Wasser ein leichtsiedendes Azeotrop bilden. Ein leichtsiedendes Azeotrop entspricht im p,x-Diagramm dem Stoffgemisch am Maximum des Dampfdrucks. Der Siedepunkt dieses Gemisches besitzt im T,x-Diagramm ein Minimum und liegt unter denjenigen der beteiligten Reinstoffe. Besonders bevorzugte organische Lösungsmittel mit einem Siedepunkt, der zwischen Wasser und EDDN oder EDMN liegt, und die mit Wasser ein leichtsiedendes Azeotrop bilden sind Toluol, N-Methylmorpholin, o-Xylol, m-Xylol oder p-Xylol, Anisol, n-Octan, n-Nonan, Diisobutylether und Diglyme, oder Mischungen davon.
Wenn das organische Lösungsmittel, das einen Siedepunkt zwischen Wasser und EDDN und/oder EDMN aufweist, ein leichtsiedendes Azeotrop mit Wasser bildet, dann ist es weiterhin bevorzugt, dass das organische Lösungsmittel eine Mischungslücke aufweist bzw. eine schlechte Löslichkeit in Wasser hat, insbesondere unter den Bedingungen der nachfolgenden beschriebenen Aufarbeitungsschritte. Hierdurch wird die spätere Trennung von Wasser und organischen Lösungsmitteln erleichtert. Vorzugsweise beträgt die Löslichkeit eines solchen organischen Lösungsmittels 1 Gew.-% oder weniger, besonders bevorzugt 0,5 Gew.-% oder weniger und insbesondere bevorzugt 0,1 Gew.-% oder weniger. Insbesondere ist Toluol als solches organisches Lösungsmittel bevorzugt.

In einer weiteren bevorzugten Ausführungsform setzt man bei der Umsetzung von von EDFA und/oder EDMFA und HCN ein organisches Lösungsmittel ein, welches einen Siedepunkt unterhalb des Siedepunkts von Wasser aufweist, und welches mit Wasser ein leichtsiedendes Azeotrop bildet, insbesondere unter den Bedingungen der nachfolgend beschriebenen destillativen Wasserabtrennung.

Besonders bevorzugte Lösungsmittel, die einen Siedepunkt unterhalb des Siedepunkts von Wasser aufweisen und die mit Wasser ein leichtsiedendes Azeotrop bilden, sind n-Pentan,n-Hexan, n-Heptan, Tetrahydrofuran, Cyclohexan, Methylcyclohexan, Leichtbenzin, Benzin (Benzol) oder Mischungen davon. Ein solches Lösungsmittel sollte unter Normalbedingungen bevorzugt einen Siedepunkt von mindestens 50°C und besonders bevorzugt von mindesten 60°C aufweisen, um so hohe Kondensationstemperaturen zu erreichen, so dass die Verwendung von Sole am Kondensator vermieden werden kann.
Es ist weiterhin bevorzugt, dass das eingesetzte Lösungsmittel, welches einen Siedepunkt unterhalb des Siedepunkts von Wassers aufweist und welches ein leichtsiedendes Azeotrop mit Wasser bildet, unter den bei der Umsetzung von von EDFA und/oder EDMFA und HCN herrschenden Bedingungen oder der nachfolgenden Aufarbeitung eine geringe

Löslichkeit in Wasser oder eine Mischungslücke mit Wasser aufweist. Hierdurch wird die spätere Trennung von Wasser und organischen Lösungsmitteln erleichtert. Vorzugsweise beträgt die Löslichkeit eines solchen organischen Lösungsmittels in Wasser 1 Gew.-% oder weniger, besonders bevorzugt 0,5 Gew.-% oder weniger und insbesondere bevorzugt 0,1 Gew.-% oder weniger.

In einer ganz besonders bevorzugten Ausführungsform wird die Umsetzung von von EDFA und/oder EDMFA und HCN zu EDDN und/oder EDMN in Gegenwart von Toluol als Lösungsmittel durchgeführt und die nachfolgende Hydrierung von EDDN und/oder EDMN zu TETA und/oder DETA wird in Gegenwart von THF durchgeführt. Wie nachfolgend beschrieben kann hierdurch ein besonders effizienter Lösungsmittelverbund eingestellt werden, der die Rückführung der organischen Lösungsmittel in das Verfahren erlaubt. Weiterhin wurde erkannt, dass die Gegenwart von THF während der nachfolgenden Hydrierung, insbesondere wenn die Hydrierung in Suspensionsfahrweise durchgeführt wird, die Agglomerationstendenz der eingesetzten Suspensionskatalysatoren verringern kann.

Demgemäß betrifft eine besonders bevorzugte Ausführungsform der vorliegenden Erfindung die Herstellung von TETA und/oder DETA durch Hydrierung von EDDN und/oder EDMN mit Wasserstoff in Gegenwart eines Katalysators, dadurch gekennzeichnet, dass die Herstellung von EDDN und/oder EDMN aus von EDFA und/oder EDMFA und HCN in Gegenwart von Toluol als Lösungsmittel erfolgt und die Hydrierung in Suspensionsfahrweise in Gegenwart von THF durchgeführt wird.

Insbesondere ist es bevorzugt, dass THF nach der EDDN und/oder EDMN-Herstellung zugeführt wird und dass nach der EDDN und/oder EDMN-Herstellung eine Behandlung von EDDN bzw. EDMN mit einem Adsorbens, bevorzugt einem festen, sauren Adsorbens, in Gegenwart von THF erfolgt.

EDFA/EDMFA

Zur Herstellung von EDFA und/oder EDMFA wird zunächst EDA mit FA umgesetzt.

In einer bevorzugten Ausführungsform wird kein organisches Lösungsmittel vor oder während der Umsetzung von EDA mit FA zu EDFA bzw. EDMFA zugeführt.

Die Umsetzung findet vorzugsweise in Gegenwart von Wasser statt, da FA, wie voranstehend beschrieben, vorzugsweise in Form von wässrigen Lösungen eingesetzt wird.

Die Umsetzung von EDA (I) mit FA zu EDFA (II) bzw. EDMFA (III) verläuft in der Regel so schnell, dass im Allgemeinen kein Katalysator benötigt wird.

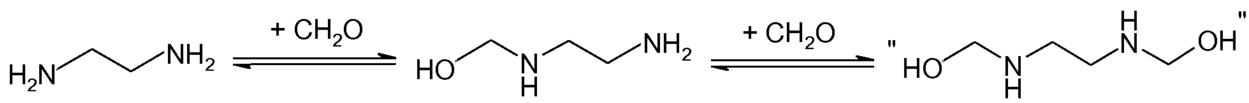


EDA (I) EDMFA (III) EDFA (II)

EDFA (II) ist formelmäßig zur besseren Übersichtlichkeit als Halbaminal dargestellt. Die Herstellung von EDFA verläuft in der Regel über das Zwischenprodukt EDMFA (III), welches aus einem Mol EDA und einem Mol Formaldehyd gebildet wird.

Die Umsetzung von EDA mit Formaldehyd zu EDFA ist im Allgemeinen stark exotherm. Die Reaktionsenthalpie liegt zwischen 100 und 120 kJ pro Mol EDA. Hinzu kommt, dass EDA mit Wasser in ebenfalls exothermer Reaktion im Allgemeinen ein Hydrat bildet. Die bei der Hydratbildung entstehende Wärmemenge stellt mit etwa 25 kJ pro Mol EDA üblicherweise rund 20 % der insgesamt frei werdenden Wärme dar.

Das Molverhältnis von EDA zu Formaldehyd beträgt 1 zu 1,8 bis 1 zu 2,2, bevorzugt 1 zu 1,9 bis 1 zu 2,1, besonders bevorzugt 1 zu 2 bis 1 zu 2,1.

Soll der EDFA-Anteil im Reaktionsgemisch erhöht werden, so beträgt das molare Verhältnis von EDA zu FA bevorzugt 1 : 1,8 bis 1 : 2,2, besonders bevorzugt 1 : 1.9 bis 1 : 2,1. Ein hoher ED-FA-Anteil im Reaktionsgemisch ist vorteilhaft, wenn EDFA in einer nachfolgenden Reaktion mit HCN zu EDDN umgesetzt wird, welches weiter zu TETA hydriert werden soll.

Soll der EDMFA-Anteil im Reaktionsgemisch erhöht werden, so beträgt das molare Verhältnis von EDA zu FA bevorzug 1 : 0,8 bis 1 : 1,5, besonders bevorzugt 1 : 1 bis 1 : 1,3. Ein höherer EDMFA-Anteil im Reaktionsgemisch ist vorteilhaft, wenn EDMFA in einer nachfolgenden Reaktion mit HCN zur EDMN umgesetzt wird, welches weiter zu DETA hydriert werden soll.

Der bei der Umsetzung von EDA mit FA eingehaltene Druck ist nicht kritisch und muss im Allgemeinen nur so hoch sein, dass der Reaktorinhalt flüssig ist. Er ist nach oben nicht begrenzt und beträgt bevorzugt 1 bis 10 bar, besonders bevorzugt 2 bis 5 bar.

Die Umsetzung von FA mit EDA erfolgt bevorzugt kontinuierlich.

Für die kontinuierliche Umsetzung von EDA mit Formaldehyd können alle für Flüssigphasen-Umsetzungen geeigneten Reaktoren verwendet werden.

Bevorzugt wird das Verfahren gemäß Option b) in einem Rohrreaktor oder einem Rührkesselreaktor oder einem Schlaufenreaktor, insbesondere einem Schlaufenreaktor, durchgeführt.

Unter einem Schlaufenreaktor ist im Folgenden ein Reaktor zu verstehen, bei dem der Reaktorinhalt im Kreis geführt wird. Der Reaktionseintrag kann nach dem Durchströmen des Reaktors in einer Kühlvorrichtung wie z. B. einem Wärmetauscher gekühlt, ein Teilstrom des gekühlten Stroms in den Reaktor zurückgeführt und der Reststrom in die nächste Verfahrensstufe geleitet werden. Dabei kann es sich um einen internen oder einen externen Kreislauf handeln. Vorzugsweise kann der externe Kreislauf in einer Kühlvorrichtung, wie z. B. einem Wärmetauscher, insbesondere Platten-, Rohrbündel- oder Doppelmantel-Wärmetauscher, gekühlt werden. Durch Ableiten der Reaktionswärme, die beispielsweise bei der Hydratation von EDA bzw. bei der Umsetzung von FA mit EDA entsteht, kann der Temperaturanstieg im Reaktor gut beherrscht werden.

Die Verweilzeit im Schlaufenreaktor beträgt bevorzugt 5 Sekunden bis 60 Minuten, besonders bevorzugt 30 Sekunden bis 20 Minuten.

Wenn die Umsetzung zu EDFA bzw. EDMFA in einem Schlaufenreaktor erfolgt, in dem eine Rückvermischung auftritt, ist der Umsatz in der Regel nicht vollständig. Er liegt im Allgemeinen im Bereich von 50 bis 99 %.

In einer ganz besonders bevorzugten Ausführungsform wird deshalb eine Kombination aus Schlaufenreaktor und nachgeschaltetem Rohrreaktor als Reaktor eingesetzt.

Dadurch kann der Umsatz, der nach Austritt aus dem Schlaufenreaktor, wie zuvor beschrieben, im Bereich von 50 bis 99% liegen kann, weiter erhöht werden.

Der nachgeschaltete Rohrreaktor wird bevorzugt unter den Bedingungen des Schlaufenreaktors, vorzugsweise bei gleichem Druck und Temperatur wie der Schlaufenreaktor betrieben

Die Edukte können vor dem Einleiten in den Reaktor oder erst im Reaktor selbst vermischt werden.

Es ist beispielsweise möglich, dass die Edukte und ggf. organische Lösungsmittel getrennt oder teilweise getrennt zugeleitet werden und die Mischung im Reaktor vorgenommen wird, beispielsweise mit Hilfe geeigneter Einbauten.

Als Mischvorrichtung sind im Allgemeinen statische Mischer, Rohrleitungen mit turbulenter Strömung, Pumpen oder Wärmetauscher geeignet.

In einer bevorzugten Ausführungsform wird das durch Mischung von EDA und FA erhaltene Gemisch in den Kreislauf des Schlaufenreaktors eingeleitet.

In einer besonders bevorzugten Ausführungsform ist eine Mischvorrichtung im Reaktorkreislauf enthalten, so dass EDA und FA über getrennte Leitungen in den Kreislauf des Reaktors eingeleitet werden können und im Kreislauf, vor der Einleitung in den Reaktorbereich, in der Mischvorrichtungen vermischt werden.

Die Temperatur bei der Umsetzung von FA und EDA zu EDFA bzw. EDMFA liegt im Allgemeinen im Bereich von 0 bis 100°C.

In einer besonders bevorzugten Ausführungsform erfolgt die Umsetzung von EDA und FA in einem engen Temperaturbereich.

Demgemäß betrifft die besonders bevorzugte Ausführungsform die

Umsetzung Ethylendiamin (EDA) mit Formaldehyd zu Ethylendiamin-Formaldehyd-Addukt (ED-FA) und/oder Ethylendiamin-Monoformaldehyd-Addukt (EDMFA), dadurch gekennzeichnet, dass die Umsetzung von FA mit EDA bei einer Temperatur im Bereich von 20 bis 50°C durchgeführt wird.

In dem Temperaturbereich der bevorzugten Ausführungsform von nur 20 bis 50 °C läuft die EDFA- bzw. EDMFA-Synthese auch ohne Katalysator schnell genug ab. Die Bildung von zwei Nebenprodukten, die bei Temperaturen von > 50 °C zu Ausbeuteverlusten führen, tritt überraschenderweise bei den Synthesetemperaturen nur in untergeordnetem Maße auf. Dabei handelt es sich um die Verbindungen IV und V, die nach der Umsetzung mit HCN identifiziert werden.

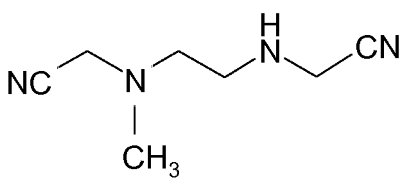

IV

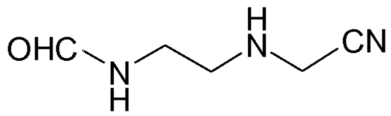

V

Es wird angenommen, dass diese Nebenprodukte auf Umsetzungen mit Formaldehyd und auf durch Cannizzaro-Reaktion entstehende Ameisensäure zurückzuführen sind. Ihr Auftreten wäre mit Wertproduktverlusten verbunden und würde zu einer Verringerung der Ausbeuten und Selektivitäten, auch in den nachfolgenden Umsetzungen von EDFA bzw. EDMFA führen. Außerdem sind die N-methylierten Nebenprodukte vom Hauptprodukt nur schwer abtrennbar, so dass sie in der Regel unerwünschte Verunreinigungen darstellen.
Das in dem Temperaturbereich von 20 bis 50 °C hergestellte Produkt aus der Umsetzung von FA und EDA weist einen geringen Anteil an den Nebenkomponenten (IV) und (V) auf, so dass die Ausbeute von EDFA und/oder EDMFA erhöht werden kann.
In der besonders bevorzugten Ausführungsform des Verfahrens gemäß Option b) liegt die Temperatur bei der Umsetzung von EDA mit FA zu EDFA und/der EDMFA im Bereich von 20 bis 50 °C, bevorzugt im Bereich von 25 bis 45°C.

Es ist weiterhin bevorzugt, dass die Umsetzung, wie zuvor beschrieben, in einem Schlaufenreaktor durchgeführt wird, insbesondere bevorzugt in der zuvor beschriebenen Kombination aus Schlaufenreaktor und Rohrreaktor.

EDDN/EDMN aus EDFA und/oder. EDMFA

EDFA und/oder. EDMFA wird anschließend weiter mit HCN zu EDDN und/oder EDMN umgesetzt.

Bevorzugt wird EDFA bzw. EDMFA ohne weitere Aufarbeitung mit HCN umgesetzt.

Das Molverhältnis von EDFA zu Blausäure (HCN) beträgt bevorzugt 1 : 1,8 bis 1 : 2,2, besonders bevorzugt 1 : 1,9 bis 1 : 2,0.

Das Molverhältnis von EDMFA zu Blausäure beträgt bevorzugt 1 : 1 bis 1 : 1,3, besonders bevorzugt 1 : 1 bis 1 : 1,2.

Die Umsetzung von EDFA bzw. EDMFA mit HCN erfolgt bevorzugt in Gegenwart eines der zuvor genannten organischen Lösungsmittel, insbesondere den bevorzugt und besonders bevorzugte genannten organischen Lösungsmitteln, durchgeführt. Die Menge an eingesetztem Lösungsmittel beträgt - wie zuvor beschrieben im Allgemeinen 0,5 bis 50 kg pro kg, bevorzugt 1 bis 30 kg, besonders bevorzugt 3 bis 25 kg pro kg eingesetztem EDA. Besonders bevorzugt erfolgt auch die Umsetzung von EDFA bzw. EDMFA mit HCN in Gegenwart von Toluol.

Der Reaktionsdruck bei der Umsetzung von HCN mit EDFA bzw. EDMFA ist in der Regel nicht kritisch. Bevorzugt wird ein Druck eingestellt, bei dem die Edukte und ggf. eingesetztes Lösungsmittel in der Flüssigphase vorliegen. Der Druck beträgt deshalb bevorzugt 1 bar bis 10 bar, besonders bevorzugt 1 bis 5 bar und insbesondere bevorzugt 1 bis 3 bar. Bevorzugt entspricht der Druck dem Druck, der bei der ggf. zuvor erfolgten Umsetzung von FA mit EDA zu EDFA bzw. EDMFA eingestellt wurde.

EDFA und/oder EDMFA und HCN und ggf. das oder die eingesetzten organischen Lösungsmittel und ggf. Wasser können vor dem Einleiten in den Reaktor oder erst im Reaktor selbst vermischt werden.

Die Umsetzung erfolgt bevorzugt in einem Rohrreaktor oder einer Rührkesselkaskade unter adiabaten Bedingungen, d.h. in einem Reaktor, der im Wesentlichen nicht gekühlt wird und die Reaktionstemperatur durch die freiwerdende Reaktionswärme erhöht wird.

Aufgrund der Exothermie der Reaktion zwischen EDFA bzw. EDMFA und HCN tritt das Reaktionsgemisch im Allgemeinen bei einer Temperatur aus dem Reaktor aus, die oberhalb der Eintrittstemperatur liegt.

Vorzugsweise wird das Reaktionsgemisch am Ausgang des Reaktors gekühlt. Die Kühlung des Reaktionsgemischs kann, wie voranstehend und im Folgenden näher beschrieben, durchgeführt werden.

Erfindungsgemäß erfolgt die Umsetzung von EDFA bzw. EDMFA mit HCN in einem Reaktor mit begrenzter Rückvermischung bei einer Temperatur im Bereich von 20 bis 120°C und kurzer Verweilzeit.
Demgemäß betrifft diesvorliegende Erfindung die
Umsetzung von Ethylendiamin-Formaldehyd-Addukt (EDFA) und/oder Ethylendiamin-Monoformaldehyd-Addukt (EDMFA) mit Blausäure (HCN) in einem Reaktor mit begrenzter Rückvermischung bei einer Temperatur im Bereich von 20 bis 120°C, dadurch gekennzeichnet, dass die Verweilzeit im Reaktor 300 Sekunden oder weniger beträgt.

Beispiele für einen Reaktor mit begrenzter Rückvermischung sind ein Rohreaktor und eine Rührkesselkaskade. Besonders bevorzugt wird die Umsetzung in einem Rohrreaktor ("plug flow reactor") durchgeführt.
Das Verhältnis von Höhe zu Durchmesser des Rohrreaktors beträgt bevorzugt 1:1 bis 500:1, besonders bevorzugt 2:1 bis 100:1 und insbesondere bevorzugt 5:1 bis 50:1.
Der Rohrreaktor kann interne Einbauten enthalten, die einer Rückvermischung in Längsrichtung entgegenwirken. Bei

den Einbauten kann es sich beispielsweise um Kugeln, Blenden, Siebböden oder statische Mischer handeln.
Ganz besonders bevorzugt wird als Rohrreaktor ein Leerrohr verwendet.
Die Lage des Reaktors ist unerheblich. Er kann senkrecht oder waagerecht sein, oder als Spirale oder Schlauder ausgeführt werden.
Bei der Umsetzung von EDFA bzw. EDMFA mit HCN beträgt die Verweilzeit im Reaktor im beanspruchten Temperaturbereich 300 Sekunden oder weniger, bevorzugt 200 Sekunden oder weniger, besonders bevorzugt 100 Sekunden oder weniger und insbesondere bevorzugt 60 Sekunden oder weniger.
In einer besonderen Ausführungsform liegt die Verweilzeit im Bereich von 1 bis 300 Sekunden, besonders bevorzugt 5 bis 200 Sekunden, ganz besonders bevorzugt 10 bis 100 Sekunden und insbesondere bevorzugt 15 bis 60 Sekunden.
Im Rahmen der vorliegenden Erfindung ist die Verweilzeit $\tau$ definiert als Quotient aus Reaktorvolumen $V_R$ und austretendem Volumenstrom $\dot{V}$ ($\tau = V_R / \dot{V}$), wobei das Reaktorvolumen das Volumen vom Reaktoreingang bis zum Reaktorausgang umfasst. Im Rahmen der vorliegenden Erfindung entspricht der Reaktoreingang der Einmischstelle, an der EDFA bzw. EDMFA mit HCN in Kontakt gebracht werden.
Im Rahmen der vorliegenden Erfindung entspricht der Reaktorausgang der Stelle, an der die Temperatur des Reaktionsgemisches durch Kühlung abgesenkt wird.
Die Kühlung des Reaktionsgemisches am Reaktorausgang kann, wie nachfolgend beschrieben, vorzugsweise durch

- Abfuhr von Wärme mittels eines Wärmetauschers,
- Zufuhr eines organischen Lösungsmittels, oder
- Entspannungsverdampfung erfolgen.

Im ersten Falle entspricht der Reaktorausgang der Stelle, an der das Reaktionsgemisch zur Kühlung in den Wärmetauscher eintritt.
Im zweiten Falle entspricht der Reaktorausgang der letzten Einmischstelle am Ausgang des Reaktors, an der weiteres organisches Lösungsmittel zur Kühlung zugeführt wird.
Im dritten Falle entspricht der Reaktorausgang dem Entspannungsventil, nach dem das Reaktionsgemisch, wie nachfolgend beschrieben, partiell verdampft wird.
Somit umfasst das Reaktorvolumen auch die Teile der Rohr- bzw. Zuleitungen zum Reaktor, die zwischen dem Reaktoreingang (Einmischstelle, an der EDFA bzw. EDMFA mit HCN in Kontakt gebracht werden) und dem Reaktorausgang (z.B. Entspannungsventil, Eingang zum Wärmetauscher oder der letzten Einmischstelle am Ausgang, an der organisches Lösungsmittel zur Kühlung zugeführt wird) liegen.

Der EDFA- bzw. FACH-haltige Strom und der HCN-Strom werden in der besonders bevorzugten Ausführungsform am Eingang des Reaktors vermischt. Die Vermischung kann mittels statischer Mischer, geeigneter Einbauten, wie Füllkörper, insbesondere Raschig-Ringe, oder durch das Erzeugen einer turbulenten Strömung an und nach der Einmischstelle erfolgen.

Die Umsetzung von EDFA bzw. EDMFA mit HCN liegt in dieser besonders bevorzugten Ausführungsform im Temperaturbereich von 20 bis 120°C, bevorzugt 25 bis 100°C und besonders bevorzugt im Bereich von 30 bis 90°C.
Besonders bevorzugt erfolgt die Umsetzung von EDFA bzw. EDMFA mit HCN in der besonders bevorzugten Ausführungsform unter adiabatischen Bedingungen, d.h. die Reaktionstemperatur im Reaktor wird durch die freiwerdende Reaktionswärme erhöht.
Im Rahmen der bevorzugten Ausführungsform sollte beachtet werden, dass die Reaktionstemperatur nicht 120°C übersteigt, da im Rahmen dieser Erfindung oberhalb dieser Temperatur eine erhöhte Zersetzung der Zielprodukte EDDN bzw. EDMN beobachtet wurde.

Um den Temperaturanstieg im Reaktor zu begrenzen, können mehrere technische Maßnahmen durchgeführt werden:

- die Edukte und ggf. organisches Lösungsmittel und ggf. Wasser können vor dem Einleiten in den Reaktor auf Temperaturen im Bereich von 10 bis 50°C, bevorzugt 20 bis 40°C und besonders bevorzugt 25 bis 35°C gekühlt werden;

- der Reaktor oder ein Teil des Reaktors kann mit Kühlvorrichtungen versehen sein; oder

- dem Reaktionsgemisch kann ein organisches Lösungsmittel zugeführt werden

Es können auch ein oder mehrere der oben genannten Maßnahmen in Kombination durchgeführt werden.

Die Edukte, sowie ggf. organisches Lösungsmittel und Wasser können bei einer Temperatur im Bereich von 10 bis 50°C, bevorzugt 15 bis 40°C und besonders bevorzugt 20 bis 35°C in den Reaktor eingeleitet werden. Sollte die Temperatur der Edukte oberhalb dieser bevorzugten Bereiche liegen, so können die Edukte mit geeigneten Kühlvorrichtungen heruntergekühlt werden, beispielsweise Wärmetauschern, insbesondere Platten-, Rohrbündel- oder Doppelmantelwärmetauschern.

Der Reaktor oder ein Teil des Reaktors kann alternativ oder zusätzlich mit Kühlvorrichtungen versehen sein. Beispielsweise kann der Reaktor eine Mantelkühlung aufweisen. Es ist auch möglich, dass sich im Reaktor Elemente befinden, die Wärme ableiten können, beispielsweise innen liegende Wärmetauscher. Weiterhin ist es auch denkbar, dass ein Teil des Reaktorinhalts über eine Schlaufe geführt wird, in der sich ein Wärmetauscher befindet. Zusätzliche Kühlvorrichtungen bedeuten jedoch in der Regel einen höheren apparativen- und Konstruktionsaufwand, diese sind aber auch geeignet, um die Temperatur im Reaktor im Bereich der besonders bevorzugten Ausführungsform zuhalten.

In einer weiteren Ausführungsform kann das Reaktionsgemisch gekühlt werden, in dem vor oder während der Umsetzung weiteres organisches Lösungsmittel zugeführt wird. Die Gesamtmenge an organischem Lösungsmittel sollte aber vorzugsweise nicht oberhalb von 50 kg pro kg EDA, bevorzugt 30 und besonders bevorzugt 25 kg pro kg EDA liegen. Bevorzugt wird das organische Lösungsmittel zur Kühlung mit einer Temperatur im Bereich von 10 bis 50°C, bevorzugt 15 bis 40°C und besonders bevorzugt 20 bis 35°C in den Reaktor eingeleitet.

Aufgrund der Exothermie der Reaktion zwischen EDFA bzw. EDMFA und HCN tritt das Reaktionsgemisch im Allgemeinen bei einer Temperatur aus dem Reaktor aus, die oberhalb der Eintrittstemperatur liegt.

Durch die Vornahme der voranstehend genannten Maßnahmen, insbesondere durch die Zugabe von organischem Lösungsmittel, kann die Austrittstemperaturen im Bereich von 50 bis 120°C, bevorzugt im Bereich von 60 bis 110°C und besonders bevorzugt im Bereich von 70 bis 100°C gehalten werden. Insbesondere ist es bevorzugt, wenn die Kühlung sowohl durch Zugabe von organischem Lösungsmittel als auch durch Kühlung des Rohrreaktors über einen Kühlmantel erfolgt.

In der ganz besonders bevorzugten Ausführungsform wird das Reaktionsgemisch am Ausgang des Reaktors zusätzlich gekühlt. Die Kühlung des Reaktionsgemischs kann, beispielsweise durch Abkühlung mittels geeigneter Kühlungsvorrichtungen, Zuführung von weiterem organischem Lösungsmittel oder durch Entspannungsverdampfung erfolgen. Die Abkühlung des Reaktionsgemischs am Ausgang des Reaktors wird nachfolgend näher beschrieben.

Reaktionsaustrag

Als Reaktionsaustrag fällt in der Regel ein Gemisch von EDDN und EDMN an.

Das Verhältnis von EDDN zu EDMN ist wie voranstehend beschrieben, im Allgemeinen durch das Verhältnis der eingesetzten Edukte beeinflussbar.

Das Gewichtsverhältnis von EDDN zu EDMN beträgt im Allgemeinen 30 : 70 bis 95 : 5, bevorzugt 50 : 50 bis 95 : 5, besonders bevorzugt 75 : 25 bis 90 : 10

Der Reaktionsaustrag kann ggf. organisches Lösungsmittel enthalten.
Vorzugsweise enthält der Reaktionsaustrag eines der zuvor genannten bzw. bevorzugt und besonders bevorzugt genannten organischen Lösungsmittel. Insbesondere enthält der Reaktionsaustrag Toluol.

Besonders bevorzugt enthält der Reaktionsaustrag 5 bis 30 Gew.-% und ganz besonders bevorzugt 10 bis 20 Gew.-% und insbesondere bevorzugt 12 bis 18 Gew.-% Toluol, bezogen auf den Reaktionsaustrag. Insbesondere bevorzugt enthält der Reaktionsaustrag neben Toluol im Wesentlichen keine weiteren organischen Lösungsmittel.

Der Reaktionsaustrag enthält im Allgemeinen Wasser, welches bei der Umsetzung von FA, HCN und EDA als Reaktionswasser entsteht oder welches zusammen mit den Edukten oder separat zugeführt wurde.

Der Reaktionsaustrag, der bei der Herstellung von EDDN bzw. EDMN anfällt, kann
nach dem Fachmann bekannten Methoden weiter aufgearbeitet werden. Dies betrifft beispielsweise die Abtrennung des Reaktionsproduktes von nichtumgesetztem Edukt sowie gegebenenfalls vorhandenem Lösungsmittel.

Kühlung des Austrags aus der Umsetzung

In einer besonders bevorzugten Ausführungsform wird das Reaktionsgemisch aus der Umsetzung von EDFA und/oder EDMFA mit HCN nach Austritt aus dem Reaktor und vor einer Aufarbeitung gekühlt.
Demgemäß betrifft die besonders bevorzugte Ausführungsform die
Herstellung von EDDN und/oder EDMN durch Umsetzung von EDFA und/oder EDMFA mit HCN, wobei die Umsetzung in Gegenwart von Wasser erfolgt, dadurch gekennzeichnet, dass das Reaktionsgemisch aus der Umsetzung von EDFA und/oder EDMFA mit HCN nach Austritt aus den Reaktor gekühlt wird.

Eine Kühlung des Reaktionsgemischs aus der Umsetzung von EDFA und/oder EDMFA und HCN ist insbesondere dann bevorzugt, wenn die letzte Stufe der Umsetzung in einem adiabat betriebenen Reaktor, insbesondere einem Rohreaktor, durchgeführt wurde.

Es ist weiterhin bevorzugt, dass die Temperatur nach dem Abkühlen im Bereich von 20 bis 70°C, besonders bevorzugt im Bereich von 20 bis 60°C und insbesondere bevorzugt im Bereich von 30 bis 50°C liegt. Durch eine schnelle Abkühlung auf den genannten Temperaturbereich ist es möglich, dass unerwünschte Nebenprodukte, beispielsweise Zersetzungsprodukte der Nitrile, weiter verringert werden.
Die Abkühlung des Reaktionsgemischs kann mittels geeigneter Kühlungsvorrichtungen, wie Wärmetauscher, insbesondere Platten-, Rohrbündel- oder Doppelmantelwärmetauscher, erfolgen.
Es ist auch möglich, dass zur Kühlung weiteres organisches Lösungsmittel zugeführt wird. Wie zuvor erwähnt, sollte die Gesamtmenge an organischem Lösungsmittel aber vorzugsweise nicht oberhalb von 50 kg pro kg EDA, bevorzugt 30 und besonders bevorzugt 25 kg pro kg EDA liegen. Bevorzugt wird das organische Lösungsmittel zur Kühlung mit einer Temperatur im Bereich von 10 bis 50°C, bevorzugt 15 bis 40°C und besonders bevorzugt 20 bis 35°C in den Reaktor eingeleitet.

Die Abkühlung erfolgt ganz besonders bevorzugt durch Entspannungsverdampfung.
Dazu wird üblicherweise das Reaktionsgemisch aus der EDDN- bzw. EDMN-Herstellung über ein Ventil am Ausgang des letzten Reaktors, in dem die EDDN bzw. EDMN-Herstellung erfolgt, in einen Behälter mit vermindertem Druck entspannt. Der verminderte Druck wird vorzugsweise so eingestellt, dass ein Teil des eingesetzten Wassers und der leichter als EDDN bzw. EDMN siedenden Komponenten im Reaktionsaustrag in die Gasphase überführt werden und die Edukte, wie EDNN bzw. EDMN, sowie ein Teil des Wassers, sowie ggf. organisches Lösungsmittel in der flüssigen Phase verbleiben.
Durch die Entspannungsverdampfung wird ein Teil des Wassers aus dem Reaktionsgemisch auf schonende Art und Weise entfernt. Durch die entzogene Verdampfungswärme wird die flüssige EDDN- bzw. EDMN-haltige Phase gekühlt. Durch beide Effekte, Abkühlung und Verringerung der Wasserkonzentration, wird das enthaltene EDDN- bzw. EDMN in der Regel stabilisiert. Nebenreaktionen werden im Allgemeinen dadurch verringert.
Vorzugsweise werden 10 bis 80 Gew.-%, besonders bevorzugt 20 bis 70 Gew.-% und ganz besonders bevorzugt 30 bis 60 Gew.-% des im Reaktionsgemisch vorhandenen Wasser bei der Entspannungsverdampfung verdampft und in die Gasphase überführt.
Bevorzugt beträgt der verminderte Druck 1000 mbar und weniger, besonders bevorzugt 300 mbar und weniger und ganz besonders bevorzugt 200 mbar und weniger.

In einer bevorzugten Ausführungsform beträgt der verminderte Druck 10 bis 1000 mbar, bevorzugt 50 bis 300 mbar und besonders bevorzugt 100 bis 200 mbar.
Der Anteil der Komponenten, die nach der Entspannungsverdampfung gasförmig vorliegen wird bevorzugt in einem Kühler partiell kondensiert, wobei die Kondensation bevorzugt so betrieben wird, dass Wasser und ggf. eingesetztes Lösungsmittel im Wesentlichen vollständig kondensiert werden. Leichter siedende Komponenten, wie z.B. Ammoniak, HCN, Methanol oder $CO_2$, werden vorzugsweise nicht kondensiert und können gasförmig entfernt bzw. einer Verbrennung zugeführt werden.

Die Aufarbeitung der kondensierten Phase kann sich danach richten, ob die Umsetzung von EDA mit HCN und FA in Gegenwart eines organischen Lösungsmittels durchgeführt wurde und danach, welches organische Lösungsmittel verwendet wurde.
Wird bei der Herstellung von EDDN bzw. EDMN kein organisches Lösungsmittel eingesetzt, so kann man das wässrige Kondensat der nachfolgend beschriebenen Kolonne K2 zuführen, in der Leichtsieder von Wasser getrennt werden. Es ist auch möglich, das Wasser einer Entsorgung zuzuführen, beispielsweise einer Abwasseraufbereitung.

Wird ein organisches Lösungsmittel eingesetzt, das mit Wasser mischbar ist bzw. keine Mischungslücke mit Wasser aufweist, so wird das kondensierte Gemisch aus organischem Lösungsmittel und Wasser in der Regel destillativ in einen wässrigen Strom und einen lösungsmittelhaltigen Strom aufgetrennt, wobei der lösungsmittelhaltigen Strom bevorzugt in das Verfahren zurückgeführt wird oder in eine nachfolgend beschriebene Kolonne K1 eingeleitet werden kann. Der wässrige Strom kann im Allgemeinen in eine Wasseraufbereitung eingeleitet werden.

Wird in einer bevorzugten Ausführungsform als organisches Lösungsmittel ein Lösungsmittel eingesetzt, welches eine Mischungslücke mit Wasser aufweist bzw. welches in Wasser im Wesentlichen nicht löslich ist, so wird das kondensierte Gemisch bevorzugt einem Phasenscheider zugeführt, so dass die kondensierte Phase in eine Phase, die das organische Lösungsmittel enthält, und eine wässrige Phase aufgetrennt werden kann.
Durch den Einsatz eines organischen Lösungsmittels, das eine Mischungslücke mit Wasser aufweist bzw. im Wesentlichen nicht in Wasser löslich ist, kann die Trennung von organischem Lösungsmittel und Wasser im Allgemeinen ohne zusätzliche Destillation erfolgen. Außerdem kann das abgetrennte Wasser nach der Phasentrennung dann im Allgemeinen direkt in eine Kläranlage eingeleitet werden oder in das Verfahren zurück geführt werden, beispielsweise zur Vermischung von EDA mit Wasser.
In dieser Hinsicht sind organische Lösungsmittel, bei denen die in der wässrigen Phase gelöste Menge an Lösungsmittel sehr niedrig ist (vorzugsweise kleiner als 5000 ppm), besonders bevorzugt. Beispiele hierfür sind Toluol, Cyclohexan, Metyhlcyclohexan, Octan, Heptan und Xylole.
Die nach der Phasentrennung erhaltene wässrige Phase kann auch in eine Destillationsvorrichtung K2 eingeleitet werden,

in der Wasser als Sumpfprodukt von leichter siedenden organischen Komponenten abgetrennt wird. Das so abgetrennte Wasser kann beispielsweise als Lösungsmittel in das Verfahren zurückgeführt (beispielsweise zur Herstellung einer wässrigen EDA-Lösung) oder einer Kläranlage oder einer biologischen Abwasseraufbereitung zugeführt werden. Die bei der Destillation in der Kolonne K2 über Kopf abgetrennten organischen Leichtsieder (beispielsweise leichter als Wasser siedende organische Lösungsmittel oder Lösungsmittel, die mit Wasser ein leichtsiedendes Azeotrop bilden, HCN oder Toluol) werden bevorzugt in den Prozess zurückgeführt. Beispielsweise können die organischen Leichtsieder dem der Entspannungsverdampfung nach geschalteten Kondensator zugeführt werden.
Die nach der Phasentrennung erhaltene organische Phase wird bevorzugt in die nachfolgend beschriebene Kolonne K1 geleitet oder als organisches Lösungsmittel in den Prozess zurückgeführt.

Der EDDN- bzw. EDMN-enthaltende Reaktionsautrag, der nach nach der Entspannungsverdampfung in den Behälter mit vermindertem Druck in der flüssigen Phase vorliegt, wird bevorzugt, wie unten beschrieben, einer Destillationskolonne K1 zugeführt, in der Wasser von EDDN bzw. EDMN abgereichert wird.
Wenn ein organisches Lösungsmittel bei der EDDN- bzw. EDMN-Herstellung eingesetzt wurde, welches unter den Bedingungen der EDDN- bzw. EDMN-Herstellung eine Mischungslücke mit Wasser bzw. in Wasser ein geringe Löslichkeit aufweist, so bilden sich im Behälter, in dem der Austrag aus der EDDN- bzw- EDMN-Herstellung entspannt wurde, üblicherweise zwei flüssige Phasen, nämlich eine wässrige EDDN- bzw. EDMN-Phase und eine Phase, die das organische Lösungsmitte enthält.
Bevorzugt werden die beiden Phasen, wie nachfolgend beschrieben, getrennt oder zusammen einer Kolonnen K1 zugeführt. Es ist weiterhin bevorzugt, dass wenn die Kolonne K1 Füllkörper enthält, beide flüssigen Phasen getrennt voneinander auf separate Flüssigkeitsverteiler zu führen.

Nach der Abkühlung kann der Reaktionsaustrag, der bei der Herstellung von EDDN bzw. EDMN anfällt, nach dem Fachmann bekannten Methoden weiter aufgearbeitet werden. Dies betrifft beispielsweise die Abtrennung des Reaktionsproduktes von nichtumgesetztem Edukt sowie gegebenenfalls vorhandenem Lösungsmittel.

Aufarbeitung des Reaktionsaustrags aus der EDDN- bzw. EDMN-Herstellung

Wie voranstehend erwähnt, kann der Reaktionsaustrag, der bei der Herstellung von EDDN bzw. EDMN anfällt, nach dem Fachmann bekannten Methoden weiter aufgearbeitet werden. Dies betrifft beispielsweise die Abtrennung des Reaktionsproduktes von nichtumgesetztem Edukt sowie gegebenenfalls vorhandenem Lösungsmittel.

Vorzugsweise wird der Reaktionsaustrag aus der EDDN- bzw. EDMN-Herstellung aufgearbeitet in dem zunächst i) eine Leichtsieder-Abtrennung und anschließend ii) eine Wasserabreicherung durchgeführt wird.

Leichtsiederabreicherung

Die Abreicherung der Leichtsieder erfolgt bevorzugt durch Strippung. So kann beispielsweise der Reaktionsaustrag aus der EDDN- bzw. EDMN-Herstellung mit Stickstoff gestrippt werden, um Spuren von Blausäure, die beispielsweise als Zersetzungsprodukt von FACH auftreten können, zu entfernen.
Die Abtrennung von Leichtsiedern kann aber auch durch Destillation erfolgen. Wenn die Abtrennung von Leichtsiedern destillativ erfolgt, dann ist es bevorzugt, dass die Verweilzeit bei der Destillation kurz gehalten wird, beispielsweise in dem die Destillation in einem Fallfilmverdampfer oder Wischfilmverdampfer durchgeführt wird.
Bevorzugt erfolgt die Leichtsieder-Abtrennung, wie zuvor beschrieben, durch Entspannungsverdampfung. Die Entspannungsverdampfung hat den Vorteil, dass die Leichtsiederabtrennung und die Kühlung des Reaktionsaustrags in einem Verfahrensschritt erfolgen können.

Wasserabreicherung

Die Wasserabreicherung nach der Abreicherung von Leichtsiedern erfolgt bevorzugt in einer Destillationskolonne K1.

Die Kolonne wird im Allgemeinen so betrieben, dass am Kopf der Kolonne ein wässriger Strom abgezogen wird, während am Kolonnensumpf ein EDDN- bzw. EDMN-haltiger Strom abgezogen wird.

Der Austrag aus der EDDN bzw. EDMN-Herstellung wird vorzugsweise zusammen mit dem Destillationsmittel (wie nachfolgend definiert) in den oberen Bereich, vorzugsweise am Kopf, einer Destillationskolonne K1 zugeführt.
Wenn der Austrag aus der EDDN bzw. EDMN-Herstellung durch Entspannungsverdampfung gekühlt wurde und wenn ein organisches Lösungsmittel bei der EDDN- bzw. EDMN-Herstellung eingesetzt wurde, welches unter den Bedingungen der EDDN- bzw. EDMN-Herstellung eine Mischungslücke mit Wasser bzw. in Wasser ein geringe Löslichkeit aufweist, so bilden sich wie zuvor beschrieben, im Behälter, in dem der Austrag aus der EDDN- bzw- EDMN-Herstellung entspannt wurde, zwei flüssige Phasen. In diesem Falle ist es bevorzugt, dass die sich bildende wässrige EDDN- bzw. EDMN-Phase und das organische Lösungsmittel als Destillationsmittel getrennt voneinander der Kolonne K1 zugeführt werden. Es ist weiterhin bevorzugt, dass wenn die Kolonne K1 Füllkörper enthält, beide flüssigen Phasen auf voneinander getrennte Flüssigkeitsverteiler zu führen. Dabei ist es bevorzugt, das organische Lösungsmittel als Destillationsmittel

in das Abtriebsteil der Kolonne, vorzugsweise in den unteren Bereich der Kolonne und besonders bevorzugt in den Sumpf der Kolonne zurückzuführen. Dies hat den Vorteil, dass HCN, welches im zurückgeführtem organischen Lösungsmittel enthalten sein, mit EDMN zu EDDN umsetzen kann. Dadurch kann die Menge an ausgeschleustem HCN verringert werden.

Bevorzugt weist die Destillationskolonne K1 Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Betten vorliegen.

Die Anzahl der theoretischen Böden liegt im Allgemeinen im Bereich von 3 bis 25, vorzugsweise 5 bis 15.

Der Kopfdruck in der Kolonne K1 wird vorzugsweise so eingestellt, dass die Sumpftemperatur in den nachfolgend genannten Bereich liegt.

Es ist bevorzugt, dass die Sumpftemperatur 100°C oder weniger beträgt, da im Rahmen der vorliegenden Erfindung gefunden wurde, dass EDMN bzw. EDDN in Gegenwart von Wasser bei höheren Temperaturen instabil ist und sich zu unerwünschten Nebenprodukten zersetzt. Bevorzugt wird eine Sumpftemperatur im Bereich von weniger als 100°C, besonders bevorzugt weniger als 80°C und ganz besonders bevorzugt weniger als 60°C eingestellt. Besonders bevorzugt liegt die Sumpftemperatur im Bereich von 20 bis 100°C, besonders bevorzugt im Bereich von 30 bis 80°C und ganz besonders bevorzugt im Bereich von 40 bis 60°C.

Der Kopfdruck beträgt bevorzugt 10 mbar bis 1 bar, besonders bevorzugt 30 mbar bis700 mbar und ganz besonders bevorzugt 50 bis 500 mbar.

In einer ganz besonderen Ausführungsform beträgt der Kopfdruck in der Kolonne K1 weniger als 300 mbar, besonders bevorzugt 100 bis 200 mbar und ganz besonders bevorzugt 130 bis 180 mbar. Im Rahmen dieser Erfindung wurde erkannt, dass sich bei den bei diesen Kopfdrücken in der Kolonne einstellenden Temperaturen, die Bildung von Ablagerungen in den Kolonneneinbauten, insbesondere den Kolonnenpackungen, Wesentlich verringert werden kann.

In einer ganz besonders bevorzugten Ausführungsform wird die Destillation in Gegenwart eines organischen Lösungsmittels durchgeführt, welches bei dem in der Kolonne herrschenden Destillationsdruck einen Siedepunkt zwischen Wasser und EDDN und/oder EDMN aufweist oder welches ein leichtsiedendes Azeotrop mit Wasser bildet.

Das organische Lösungsmittel, welches bei dem in der Kolonne herrschenden Destillationsdruck einen Siedepunkt zwischen Wasser und EDDN und/oder EDMN aufweist, oder welches ein leichtsiedendes Azeotrop mit Wasser bildet, wird nachfolgend als Destillationsmittel bezeichnet.

Bevorzugte Destillationsmittel sind die Eingangs genannten organischen Lösungsmittel, die einen Siedepunkt zwischen Wasser und EDDN und/oder EDMN aufweisen oder welche ein leichtsiedendes Azeotrop mit Wasser bilden.

Ganz besonders bevorzugt wird als Destillationsmittels Toluol eingesetzt.

Es ist bevorzugt, dass das Destillationsmittel bereits vor oder während der Umsetzung von FA, HCN und EDA zugeführt wird. Wir voranstehend erwähnt, beträgt die Menge an organischem Lösungsmittel im Allgemeinen 0,1 bis 50 kg pro kg, bevorzugt 1 bis 30 kg, besonders bevorzugt 3 bis 25 kg pro kg eingesetztem EDA.

Die Menge Destillationsmittel sollte in der Regel so bemessen sein, dass im Kolonnensumpf der Destillationskolonne K1 - wie voranstehend beschrieben--, bevorzugt eine Sumpftemperatur im Bereich von weniger als 100°C, besonders bevorzugt weniger als 80°C und ganz besonders bevorzugt weniger als 70°C und insbesondere bevorzugt weniger als 60°C eingestellt wird. Bevorzugt liegt die Sumpftemperatur im Bereich von 20 bis 100°C, besonders bevorzugt im Bereich von 30 bis 80°C und ganz besonders bevorzugt im Bereich von 40 bis 60°C.

Es ist bevorzugt, dass die Sumpftemperatur 100°C oder weniger beträgt, da im Rahmen der vorliegenden Erfindung gefunden wurde, dass EDMN bzw. EDDN in Gegenwart von Wasser bei höheren Temperaturen instabil ist und sich zu unerwünschten Nebenprodukten zersetzt. Wenn das Destillationsmittel mit Wasser ein leichtsiedendes Azeotrop bildet, dann ist erforderlich, dass die Menge an Destillationsmittel so groß ist, dass man auf der richtigen "Seite" des Azeotropes liegt, d.h. das die Menge an Destillationsmittel so groß sein muss, dass am Kopf der Kolonne das leichtsiedende, wässrige Azeotrop und im Sumpf der Kolonne im Wesentlichen kein Wasser mehr anfällt. Die erforderliche Lösungsmittelmenge kann vom Fachmann, in Abhängigkeit des gewählten Destillationsmittels, aus allgemein bekannten Tabellen und Nachschlagewerken für Azeotrope, routinemäßig ermittelt werden.

Der Kopfdruck in der Kolonne K1 beträgt, wie zuvor beschrieben, bevorzugt 10 mbar bis 1 bar, besonders bevorzugt 30 mbar bis 700 mbar und ganz besonders bevorzugt 50 bis 500 mbar. In einer ganz besonderen Ausführungsform beträgt der Kopfdruck in der Kolonne K1 weniger als 300 mbar, besonders bevorzugt 100 bis 200 mbar und ganz besonders bevorzugt 130 bis 180 mbar. Im Rahmen dieser Erfindung wurde erkannt, dass sich bei den bei diesen Kopfdrücken in der Kolonne einstellenden Temperaturen, die Bildung von Ablagerungen in den Kolonneneinbauten, insbesondere den Kolonnenpackungen, wesentlich verringert werden kann.

Der Kondensator der Destillationskolonne K1 wird im Allgemeinen bei einer Temperatur betrieben, in dem der

überwiegende Teil des Wassers oder des Wasser-Azeotrop bei dem entsprechenden Kopfdruck kondensiert wird. In der Regel liegt die Betriebstemperatur des Kondensators im Bereich von 20 bis 70°C, vorzugsweise 25 bis 50°C.
Im Kondensator fällt im Allgemeinen ein Kondensat an, welches im wesentlichen Wasser oder ein leichtsidendes Wasser-Azeotrop enthält.

Das Kondensat der Kolonne K1 kann entweder ausgeschleust werden oder in das Verfahren zurückgeführt werden. Ggf. kann das Kondensat vor der Rückführung bzw. Ausschleusung in Wasser und Destillationsmittel getrennt werden, beispielsweise durch Destillation. Beispielsweise kann die Destillation von Wasser in der zuvor beschriebenen Kolonne K2 durchgeführt werden.
Weist das Destillationsmittel eine Mischungslücke mit Wasser auf, so kann die Trennung von Wasser und Destillationsmittel auch mittels Phasentrennung erfolgen.

In einer bevorzugten Ausführungsform werden die Brüden vom Kopf der Kolonne K1 dem Kondensator zugeführt, an dem die Brüden kondensiert werden, die bei der Entspannungsverdampfung entstehen, d.h. dass die Brüden aus der Kolonne K1 und aus der Entspannungsverdampfung auf einen gemeinsamen Kondensator gefahren werden.

Reaktionsaustrag aus der Kolonne K1

Im Sumpf der Kolonne K1 wird vorzugsweise als Sumpfprodukt ein Gemisch abgezogen, dass EDDN bzw. EDMN enthält.
Das EDDN-bzw. EDMN-haltige Gemisch enthält vorzugsweise das bei der destillativen Abreicherung von Wasser eingesetzte Destillationsmittel.
Wird als Destillationsmittel Toluol eingesetzt, so enthält das EDDN-bzw. EDMN-haltige Gemisch aus dem Sumpf der Kolonne K1 vorzugsweise 5 bis 30 Gew.-% Toluol und ganz besonders bevorzugt 10 bis 20 Gew.-% und insbesondere bevorzugt 12 bis 18 Gew.-%, bezogen auf den ausgetragenen Sumpf.

Das EDDN-bzw. EDMN-haltige Gemisch aus dem Sumpf der Kolonne K1 enthält in der bevorzugten Ausführungsform - im Gegensatz zu den im Stand der Technik beschriebenen Mengen von mehr als 10 Gew.-% - bevorzugt weniger als 3 Gew.-%, besonders bevorzugt weniger als 1 Gew.-% Wasser, ganz besonders bevorzugt weniger als 0,5 Gew.-% und insbesondere bevorzugt weniger als 0,3 Gew.-% Wasser.

Das so erhaltene EDDN- bzw. EDMN-haltige Gemisch kann direkt in einer nachfolgenden Reaktion mit Wasserstoff und in Gegenwart eines Katalysators zu DETA bzw. TETA hydriert werden.

Behandlung mit Adsorbens

In einer weiteren besonders bevorzugten Ausführungsform wird das EDDN- bzw. EDMN-haltige Gemisch nach der Wasserabreicherung jedoch vor der Hydrierung des EDDN bzw. EDMN zu TETA bzw. DETA aufgereinigt, in dem man das EDDN- bzw. EDMN-haltige Gemisch mit einem Adsorbens behandelt.

In einer ganz besonders bevorzugten Ausführungsform erfolgt die Behandlung mit einem festen, sauren Adsorbens. Im Rahmen der vorliegenden Erfindung wurde gefunden, dass mit festen, sauren Adsorbentien, die Standzeit von Hydrierkatalysatoren in der nachfolgenden Hydrierung zu DETA bzw. TETA verlängert werden kann. Weiterhin wurde gefunden, dass die Bildung der bei der Hydrierung von EDDN bzw. EDMN auftretenden Nebenrprodukte Aminoethylpiperazin (AEPIP), welche in der Regel mit dem Aktivitätsverlust des Katalysators einhergehen, verringert werden können.
Somit betrifft diese weitere besonders bevorzugte Ausführungsform die
zur Herstellung von EDDN und/oder EDMN durch

a) Umsetzung von EDFA und/oder EDMFA und HCN, wobei die Umsetzung in Gegenwart von Wasser erfolgt,
b) Wasser aus dem in Stufe a) erhaltenen Reaktionsgemisch abgereichert, und
c) das Gemisch aus Stufe b) mit einem Adsorbens in Gegenwart eines organischen Lösungsmittels behandelt wird, dadurch gekennzeichnet, dass das Adsorbens ein festes, saures Adsorbens ist.

Stufe a)

Verfahren zur Umsetzung von EDFA und/oder EDMFA und HCN in Gegenwart von Wasser (Stufe a)) sind voranstehend beschrieben worden. Beispielsweise kann die Umsetzung gemäß der zuvor beschriebenen Optionen a) bis d) erfolgen, insbesondere gemäß der als bevorzugt beschriebenen Ausführungsformen.

Stufe b)

Die Abreicherung von Wasser aus dem Reaktionsaustrag der EDDN- bzw. EDMN-Herstellung ist ebenfalls

voranstehend beschrieben.
Bevorzugt wird aus dem Reaktionsaustrag aus der EDDN- bzw. EDMN-Herstellung zunächst Leichtsieder, wie HCN oder Methanol abgetrennt, beispielweise durch Strippen oder Entspannungsverdampfung, und das Wasser enthaltende EDDN- bzw. EDMN nachfolgend einer Destillation zugeführt, in der Wasser abgereichert wird. Ganz besonders bevorzugt erfolgt die Destillation, wie zuvor beschrieben, in Gegenwart eines Destillationsmittels (Definition siehe oben).

Spezifikation: Eintrag Stufe c)

Das EDDN bzw. EDMN-Gemisch aus Stufe b) enthält bevorzugt 95 Gew.-% EDDN und/oder EDMN und mehr, besonders bevorzugt 97 Gew.-% und mehr, ganz besonders bevorzugt 99 Gew.-% und mehr, bezogen auf das EDDN-Gemisch abzüglich des im EDDN-Gemisch enthaltenden Destillationsmittel und/oder organischen Lösungsmittels ("destillationsmittelfrei und lösungsmittelfrei" gerechnet).
Wie zuvor beschrieben, enthält das aus Stufe b) erhaltene Gemisch vorzugsweise das bei der Abreicherung von Wasser eingesetzte Destillationsmittel.
Wurde als Destillationsmittel Toluol eingesetzt, so enthält das EDDN- bzw. EDMN-Gemisch aus Stufe b) vorzugsweise 5 bis 30 Gew.-% Toluol, besonders bevorzugt 10 bis 20 Gew.-% Toluol, und ganz besonders bevorzugt 12 bis 18 Gew.-%.
Wenn Destillationsmittel eingesetzt werden, die keine Mischungslücke mit EDDN aufweisen, dann enthält das EDDN- bzw. EDMN-Gemisch aus Stufe b) vorzugsweise 5 bis 50 Gew.-% EDDN und/oder EDMN, besonders bevorzugt 8 bis 30 Gew.-% EDDN und/oder EDMN, und ganz besonders bevorzugt 10 bis 20 Gew.-% EDDN und/oder EDMN.
Das aus Stufe b) erhaltene EDDN- bzw. EDMN-Gemisch enthält bevorzugt weniger als 3 Gew. %, besonders bevorzugt weniger als 1 Gew.-%, ganz besonders bevorzugt weniger als 0,5 Gew.-% Wasser und insbesondere bevorzugt weniger als 0,3 Gew.-% Wasser, bezogen auf EDDN und EDMN.

Stufe c)

In der besonders bevorzugten Ausführungsform wird in Stufe c) das aus Stufe b) erhaltene EDDN bzw. EDMN mit einem festen, sauren Adsorbens in Gegenwart eines organischen Lösungsmittels behandelt.
Als Lösungsmittel sind alle organischen Lösungsmittel geeignet, die zur Umsetzung von EDDN bzw. EDMN eingesetzt werden können. Wie voranstehend erwähnt, ist es bevorzugt, dass die eingesetzten organischen Lösungsmittel unter den Bedingungen der EDDN- bzw. EDMN-Hydrierung stabil sind.
Bevorzugt wird das organische Lösungsmittel vor der Behandlung des EDDN- bzw. EDMN-Gemischs aus Stufe b) mit dem Adsorbens zugeführt.
Bevorzugt wird soviel organisches Lösungsmittel zugeführt, dass die Konzentration von EDDN und/oder EDMN in dem Gemisch, welches mit dem Adsorbens behandelt wird, im Bereich von 5 bis 50 Gew.-%, besonders bevorzugt 8 bis 30 Gew.-% und ganz besonders bevorzugt 10 bis 20 Gew.-% beträgt.
Es ist weiterhin bevorzugt, dass der Wassergehalt von organischen Lösungsmitteln, die nach der EDDN und/oder EDMN-Herstellung und vor oder während der Behandlung des EDDN und/oder EDMN mit Adsorbens zugeführt werden, einen geringen Wassergehalt aufweisen, da gefunden wurde, dass geringe Mengen Wasser bei der Behandlung mit Adsorbens das Absorptionsvermögen des Adsorbens verringern können und bei der nachfolgenden Hydrierung von EDDN bzw. EDMN polare Verunreinigungen eingeschleppt werden können, die zu unerwünschten Nebenreaktionen führen.
Besonders bevorzugt enthält das zugeführte organische Lösungsmittel weniger als 0,5 Gew.-% Wasser, besonders bevorzugt weniger als 0,3 Gew.-% Wasser, ganz besonders bevorzugt weniger als 0,1 Gew.-% Wasser und insbesondere bevorzugt weniger als 0,03 Gew.-% Wasser.
In einer ganz besonders bevorzugten Ausführungsform wird THF als organisches Lösungsmittel zugeführt. Bei der Verwendung von THF konnten in der nachfolgenden Hydrierung besonders gute Katalysatorstandzeiten erzielt werden. Wird die nachfolgende Hydrierung in Suspensionsfahrweise durchgeführt, so kann durch den Einsatz von THF die Agglomerationstendenz von Suspensionskatalysatoren während der Hydrierung verringert werden.

Feste, saure Adsorbentien

Im Rahmen der vorliegenden Erfindung wird unter festen, sauren Adsorbens ein wasserunlösliches poröses Material verstanden, das aufgrund seiner großen Oberfläche Wasser oder andere Moleküle durch physikalische oder chemische Kräfte an sich binden kann
Ein saures Adsorbens weist in der Regel funktionelle Gruppen auf, die sich unter den Bedingungen der Adsorption als Brönstedt- oder Lewis-Säuren verhalten. Insbesondere ist ein saures Sorbens in der Lage, bevorzugt basische Stoffe zurückzuhalten gegenüber weniger basischen Stoffen.
Bevorzugte feste, saure Adsorbentien sind saure Metalloxide, wie Siliziumdioxid, Titandioxid, Aluminiumoxid, Boroxid ($B_2O_3$), Zirkondioxid, Silikate, Alumosilikate, Borosilikate, Zeolithe (insbesondere in der H-Form), saure Ionentauscher, und Silikagel, z.B. Sorbead WS der BASF SE, oder Mischungen dieser Stoffe.
Ganz besonders bevorzugte feste, saure Adsorbentien sind Siliziumdioxid und Silikagel.
Ganz besonders bevorzugt sind Silikagele, die z.B. durch Ansäuern von wässrigen Natronwasserglas-Lösungen und Trocknen der zunächst erhaltenen Kieselsole hergestellt werden können, wie beispielsweise im Hollemann-Wiberg (Lehrbuch der Anorganischen Chemie, 102. Auflage, Verlag Walter der Gruyter, 2007, Seite 962) beschrieben sind.
Beispiele für besonders bevorzugte Silikagele sind Sorbead WA der BASF SE und Silikagel KG 60 der Merck KGaA.
In einer bevorzugten Ausführungsform ist das feste, saure Adsorbens ein Stoff ausgewählt aus der Gruppe bestehend aus Siliziumdioxid, Titandioxid, Aluminiumoxid, Boroxid ($B_2O_3$), Zirkondioxid, Silikaten, Alumosilikaten, Borosilikaten, Zeolithen (insbesondere in der H-Form), sauren Ionentauschern und Silikagel.

Im Rahmen der vorliegenden Erfindung umfasst das Merkmal festes, saures Adsorbens weder Aktivkohle noch nicht-saure (basische) Ionenaustauscher.

Die Behandlung des in Stufe b) erhaltenen EDDN- bzw. EDMN-Gemischs mit organischem Lösungsmittel kann entweder kontinuierlich, semi-kontinuierlich oder diskontinuierlich erfolgen.

Die Behandlung kann diskontinuierlich erfolgen, beispielsweise in dem man das Adsorbens mit dem EDDN bzw. EDMN in Gegenwart eines organischen Lösungsmittels in Kontakt bringt. Die Behandlung kann dadurch erfolgen, dass man das Adsorbens in dem zu reinigenden Gemisch suspendiert, z.B. durch Rühren in einem geeigneten Behälter. Die Behandlungszeit bei der diskontinuierlichen Behandlung liegt in der Regel im Bereich von 1 Minute bis zu 48 Stunden, bevorzugt 5 Minuten bis 24 Stunden, besonders bevorzugt 1 Stunde bis 16 Stunden und insbesondere bevorzugt 2 bis 8 Stunden.
Die Menge an Adsorbens liegt bevorzugt im Bereich von 0,1 bis 25 Gew.-%, besonders bevorzugt im Bereich von 0,5 bis 20 Gew.-% und ganz besonders bevorzugt im Bereich von 1 bis 10 Gew.-%, bezogen auf die Summe von EDDN, EDMN und organischem Lösungsmittel.

Der Druck ist in der Regel nicht kritisch. Es ist jedoch bevorzugt einen Druck einzustellen, bei dem das zu reinigende Gemisch flüssig vorliegt. Der Druck beträgt in der Regel 1 bis 10 bar. Die Behandlung erfolgt in der Regel bei Temperaturen von weniger als 150°C, bevorzugt weniger als 100°C, besonders bevorzugt bei weniger als 80°C und insbesondere bevorzugt weniger als 60°C.
Die diskontinuierliche Behandlung mit Adsorbens kann unter einer Inertgasatmospäre erfolgen, beispielsweise unter Stickstoff oder Argon.
Nach der Behandlung kann das Adsorbens durch geeignete Verfahren von EDDN bzw. EDMN abgetrennt werden, beispielsweise durch Filtration, Zentrifugation oder Sedimentation.

Bevorzugt erfolgt die Behandlung des aufzureinigenden Gemischs kontinuierlich.
Besonders bevorzugt wird das zu reinigende Gemisch über ein oder mehrere Festbetten oder Schüttungen des Adsorbens geleitet. Das Adsorbens kann auch in Form eines Wirbelbettes angeordnet sein
Das Festbett oder die Schüttung ist bevorzugt in einem Rohr oder einem Wärmetauscher angeordnet.
Das Festbett bzw. die Schüttung wird im Allgemeinen von dem zu reinigenden Gemisch durchströmt.
Die Belastung beträgt bevorzugt 0,01 bis 20, besonders bevorzugt 0,05 bis 15 und ganz besonders bevorzugt 0,1 bis 10 kg zu reinigendes Gemisch pro kg Adsorbens pro Stunde. Das Festbettvolumen und die Größe der Adsorbenspartikel können in weiten Bereichen variiert und so den gewählten Reaktionsbedingungen und den Verfahrensgegebenheiten angepasst werden.

Die Partikelgröße der eingesetzten festen, sauren Adsorbentien beträgt jedoch vorzugsweise 0.1 bis 10, besonders bevorzugt 0.5 bis 6 und ganz besonders bevorzugt 1 bis 4 mm, da gefunden wurde, dass zu große Partikel negative Diffusionseffekte aufweisen und zu kleine Partikel zu Verstopfungen im Adsorber führen können. Bevorzugt sind die Partikel kugelförmig.

In einer bevorzugten Variante liegt das Adsorbens in einem Festbett in Karussell-Anordnung, insbesondere mit Regeneration, vor, d.h. es werden zwei oder mehrere Festbetten alternativ durchströmt, so dass die nicht genutzten Festbetten regeneriert werden können.
Der Druck ist in der Regel nicht kritisch. Es ist jedoch bevorzugt einen Druck einzustellen, bei dem das zu reinigenden Gemisch flüssig vorliegt. Der Druck beträgt in der Regel 1 bis 10 bar. Die Behandlung erfolgt, wie bereits zuvor beschrieben, in der Regel bei Temperaturen von weniger als 150°C, bevorzugt weniger als 100°C, besonders bevorzugt bei weniger als 80°C und insbesondere bevorzugt weniger als 60°C.
Die kontinuierliche Behandlung mit Adsorbens kann unter einer Inertgasatmospäre erfolgen, beispielsweise unter Stickstoff oder Argon.
Falls erforderlich kann nach der kontinuierlichen Behandlung das Adsorbens oder Teile des Adsorbens, z.B. Abrieb, durch geeignete Verfahren von EDDN bzw. EDMN abgetrennt werden, beispielsweise durch Filtration, Zentrifugation oder Sedimentation.

Es kann erforderlich sein, dass das Adsorbens nach einer gewissen Betriebsdauer regeneriert werden muss, falls die Wirkung des Adsorbens mit zunehmender Betriebsdauer nachlässt.

Die Regenerierung des Adsorbens kann durch Waschen mit Wasser, bevorzugt durch Waschen mit verdünnten

wässrigen Säuren, besonders bevorzugt zunächst durch Waschen mit Wasser und anschließend durch Waschen mit verdünnten wässrigen Säuren erfolgen. Bevorzugt werden zum Waschen verdünnte, organische Säuren eingesetzt, besonders bevorzugt Essigsäure.
Bevorzugt beträgt die Konzentration an Säuren in den verdünnten, wässrigen Säuren 10 Gew.-% oder weniger.

Vorzugsweise wird das Sorbens nach der Behandlung mit Wasser und/oder wässriger Säure durch Einleitung eines trockenen Gases wie Luft oder Stickstoff getrocknet. Bevorzugt wird bei der Trocknung mit einem trockenen Gas das Sorbens und/oder das Gas aufgewärmt.

In einer besonders bevorzugten Verfahrensvariante wird das Sorbens durch Überleiten eines trockenen organischen Lösungsmittels getrocknet. Besonders bevorzugt ist das gleiche organische Lösungsmittel, das in der nachfolgenden Hydrierung verwendet wird bzw. das bei der Behandlung mit Adsorbens bereits zugegen ist. Bevorzugt enthält das trockene organische Lösungsmittel 1 Gew.-% Wasser oder weniger, besonders bevorzugt 0,5 Gew.-% oder weniger, ganz besonders bevorzugt 0,1 Gew.-% oder weniger und insbesondere bevorzugt 0,05 Gew.-% oder weniger. Das trockene organische Lösungsmittel kann entweder flüssig oder dampfförmig über das Adsorbens geleitet werden.

Bevorzugt enthält das Gemisch aus Stufe c) EDDN und/oder EDMN zusammen mit dem organischen Lösungsmittel, in dessen Gegenwart die Behandlung mit Adsobens durchgeführt wurde, und ggf. Destillationsmittel, welches bevorzugt bei der Wasserabreicherung zugegen war. Ggf. kann das Gemisch aus Stufe c) noch weitere organische Lösungsmittel enthalten.

Der Wassergehalt des Gemischs aus Stufe c) ist vorzugsweise geringer als der Wassergehalt des EDDN- bzw. EDMN-Gemischs vor der Behandlung mit Adsorbens, da das Adsorbens auch einen Trocknungseffekt aufweist. Vorzugsweise beträgt der Wassergehalt des Gemischs aus Stufe c) vorzugsweise 0,1 Gew.-oder weniger, besonders bevorzugt 0,03 Gew.-% oder weniger.

Das aus Stufe c) erhaltene EDDN bzw. EDMN-Gemisch kann aufgereinigt werden, beispielsweise kann das ggf. zugegebene organische Lösungsmittel von EDDN bzw. EDMN abgetrennt werden.

Bevorzugt wird allerdings das aus c) erhaltene Gemisch direkt - ohne weitere Aufarbeitungder Hydrierung zugeführt.

Die Hydrierung kann, wie nachfolgend beschrieben, durchgeführt werden.

Hydrierung von EDDN bzw. EDMN zu TETA bzw. DETA

Die Hydrierung von EDDN bzw. EDMN zu TETA bzw. DETA erfolgt im Allgemeinen durch Umsetzung von EDDN bzw. EDMN mit Wasserstoff in Gegenwart eines Katalysators und eines organischen Lösungsmittels.

Die Herstellung von EDDN bzw. EDMN erfolgt bevorzugt- wie zuvor beschrieben - gemäß einer der zuvor beschriebenen Optionen a) bis d), insbesondere der dort beschriebenen bevorzugten Ausführungsformen.
Weiterhin ist es bevorzugt, dass das Reaktionsgemisch aus der EDDN- bzw. EDMN-Herstellung gekühlt wird, vorzugsweise durch Entspannungsverdampfung.
Weiterhin ist es bevorzugt, dass das Reaktionsgemisch aus der EDDN- bzw. EDMN-Herstellung aufgereinigt wird, vorzugsweise, wie beschrieben, durch Abreicherung von Leichtsiedern, vorzugsweise durch Entspannungsverdampfung, und nachfolgender Destillation zur Abreicherung von Wasser, vorzugsweise in Gegenwart eines Destillationsmittels.
Es ist weiterhin bevorzugt, dass das EDDN- bzw. EDMN-Gemisch nach der Abreicherung von Wasser mit einem Adsorbens behandelt wird, vorzugsweise, wie beschrieben mit einem festen, sauren Adorbens.

Bevorzugt enthält das Gemisch, das in die Hydrierung eingeleitet wird, EDDN und/oder EDMN. Der Anteil an EDDN und/oder EDDN in dem Gemisch, dass der Hydrierung zugeführt wird, liegt vorzugsweise im Bereich von 5 bis 50 Gew.-%, besonders bevorzugt 8 bis 30 Gew.-% und ganz besonders bevorzugt 10 bis 20 Gew.-% beträgt.

Vorzugsweise enthält das Gemisch, das in die Hydrierung eingeleitet wird, das organische Lösungsmittel, das bei der Behandlung mit Adsorbens zugegen war.

Weiterhin enthält das Gemisch, das in die Hydrierung eingeleitet wird, Destillationsmittel, welches bevorzugt bei der destillativen Wasserabreicherung eingesetzt wurde.

Wasserstoff

Die Herstellung von TETA bzw. DETA findet in Gegenwart von Wasserstoff statt.
Der Wasserstoff kommt im Allgemeinen technisch rein zum Einsatz. Der Wasserstoff kann auch in Form eines Wasserstoff enthaltenden Gases, d.h. mit Beimengungen anderer Inertgase, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr

als 99,9 Gew.-% Wasserstoff, besonders bevorzugt mehr als 99,99 Gew.-% Wasserstoff, insbesondere mehr als 99,999 Gew.-% Wasserstoff.

Organisches Lösungsmittel

Die Herstellung von TETA bzw. DETA findet bevorzugt in Gegenwart eines organischen Lösungsmittels statt. Es ist bevorzugt, dass das organische Lösungsmittel dasselbe Lösungsmittel ist, das bei der Behandlung mit Adsorbens zugegen war. Es ist allerdings auch möglich ein weiteres Lösungsmittel hinzugeben oder das Lösungsmittel, welches während der Behandlung mit Adsorbens zugegen war, abzutrennen und ein neues Lösungsmittel zuzugeben. Als organisches Lösungsmittel können alle organischen Lösungsmittel verwendet werden, die bei der Herstellung von EDDN bzw. EDMN eingesetzt werden können, insbesondere die als bevorzugt genannten organischen Lösungsmittel.
Das Gewichtsverhältnis von organischem Lösungsmittel zu EDDN bzw. EDMN während der Hydrierung beträgt bevorzugt 0,01 : 1 bis 99 : 1, besonders bevorzugt 0,05 : 1 bis 19 : 1 und ganz besonders bevorzugt 0,5 : 1 bis 9 : 1.
Es ist aber ganz besonders bevorzugt, dass die Hydrierung in Gegenwart von THF durchgeführt wird, da in THF die Agglomerationstendenz von Katalysatoren bei der Suspensionsfahrweise verringert werden kann. Besonders bevorzugt findet die Hydrierung in Gegenwart von soviel THF statt, dass der Gehalt an EDDN und/oder EDMN während der Hydrierung vorzugsweise im Bereich von 5 bis 50 Gew.-%, besonders bevorzugt 8 bis 30 Gew.-% und ganz besonders bevorzugt 10 bis 20 Gew.-% beträgt.
Es ist weiterhin bevorzugt, dass die Herstellung von EDDN und/oder EDMN, wie zuvor beschrieben, in Gegenwart von Toluol erfolgt.

Wasser

Die Hydrierung von EDDN bzw. EDMN kann auch in Gegenwart von Wasser erfolgen.
Es ist jedoch bevorzugt kein weiteres Wasser zuzuführen, da sowohl EDDN und EDMN in Gegenwart von Wasser zur Zersetzung neigen.
Bevorzugt wird ein EDDN bzw. EDMN eingesetzt, welches weniger als 3 Gew.-%, bevorzugt weniger als 1 Gew.-%, besonders bevorzugt weniger als 0,5 Gew.-% Wasser und insbesondere bevorzugt weniger als 0,3 Gew.%, bezogen auf EDDN bzw. EDMN enthält.
Ganz besonders bevorzugt wird ein EDDN bzw. EDMN eingesetzt, welches weniger als 0,1 Gew.-% und insbesondere bevorzugt weniger als 0,03 Gew.-% Wasser, bezogen auf EDDN bzw. EDMN, enthält.
Insbesondere ist es bevorzugt, dass EDDN und/oder EDMN mit einem geringen Wassergehalt durch Behandlung des EDDN und/oder EDMN mit Adsorbens erhalten wird.

Zusatzstoff: Basische Verbindungen

In einer weiteren bevorzugten Verfahrensvariante findet die Hydrierung in Gegenwart von basischen Verbindungen statt, die bevorzugt in geeigneten Lösungsmitteln, wie Alkanolen, wie C1-C4-Alkanolen, z.B. Methanol oder Ethanol, oder Ethern, wie cyclischen Ethern, z.B. THF oder Dioxan, dem Reaktionsgemisch zugefügt werden.
Besonders bevorzugt werden Lösungen von Alkali- oder Erdalkalihydroxiden oder von Hydroxiden der Seltenen Erdmetalle in Wasser, besonders bevorzugt Lösungen von LiOH, NaOH, KOH und/oder CsOH , zugegeben.
Es wird bevorzugt so viel Alkali- und/oder Erdalkalimetallhydroxid zugeführt, dass die Konzentration von Alkali- und/oder Erdalkalihydroxid bezogen auf das zu hydrierende Gemisch im Bereich von 0,005 bis 1 Gew.-%, besonders bevorzugt 0,01 bis 0,5 Gew.-% und ganz besonders bevorzugt 0,03 bis 0,1 Gew.-% liegt.
Als basische Verbindungen können aber auch bevorzugt Amide und/oder Amine, wie Ammoniak und EDA, eingesetzt werden.
Durch Zusatz solcher basischen Additive lässt sich die Menge an gebildeten Nebenprodukten, wie AEPIP, bei der Hydrierung verringern.
Bevorzugte Beispiele für derartige Additive sind Ammoniak und Ethylendiamin.
Die Menge an diesen Additiven beträgt 0,01 bis 10 Mol pro Mole EDDN + EDMN.
Die basischen Additive können im Allgemeinen diskontinuierlich oder kontinuierlich sowie vor und/oder während der Hydrierung zugeführt werden.

Katalysatoren

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Darin eingeschlossen sind so genannte oxidische Katalysatoren, die die ein oder mehrere aktive Spezies in Form ihrer sauerstoffhaltigen Verbindungen enthalten und so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

In einer besonders bevorzugten Ausführungsform werden bei Hydrierung von EDDN und/oder EDMN Raney-Katalysatoren eingesetzt, bevorzugt Raney-Kobalt- oder Raney-NickelKatalysatoren und besonders bevorzugt mit mindestens einem der Elemente Cr, Ni oder Fe dotierte Raney-Kobalt- oder mit einem der Elemente Mo, Cr oder Fe dotierte Raney-NickelKatalysatoren.

Die Katalysatoren können als Vollkatalysatoren oder geträgert eingesetzt werden. Als Träger kommen bevorzugt Metalloxide wie $Al_2O_3$, $SiO_2$, $ZrO_2$, $TiO_2$, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoff-enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% $ZrO_2$,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdäns enthalten, beispielsweise der in diesem Dokument offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$ enthält. Beispielsweise der in dieser Schrift konkret offenbarte Katalysator mit der Zusammensetzung 31,5 Gew.-% $ZrO_2$, 50 Gew.-% NiO, 17 Gew.-% CuO und 1,5 Gew.-% $MoO_3$. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die zur Hydrierung von EDDN und/oder EDMN eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmitteln gewaschen werden.

In dem Katalysator können einzelne oder mehrere weitere Elemente als Promotoren anwesend sein. Beispiele

für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der nachfolgenden Hydrierung zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgende Waschung mit Wasser erhalten wurde, und bevorzugt als Promotoren mindestens eines der Elemente Fe, Ni oder Cr enthält.

Solche bevorzugten Raney-Co-Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, besonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew.-% Ni, insbesondere 1 - 4 Gew.-% Ni, wobei die Gewichtsangaben jeweils auf das Katalysatorgesamtgewicht bezogen sind.

Als Katalysator bei der Hydrierung kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:

Al: 2-6 Gew.-%, Co: $\geq$ 86 Gew.-%, Fe: 0-1 Gew.-%, Ni: 1-4 Gew.-%, Cr: 1,5 - 3,5 Gew.-%.

Regenerierung - Allgemein

Die Katalysatoren, die bei der Umsetzung von EDDN bzw. EDMN mit Wasserstoff eingesetzt werden, können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Aus EP 892777 ist bekannt, desaktivierte Raney-Katalysatoren bei Temperaturen von 150 bis 400 °C und Drucken von 0,1 bis 30 MPa 2 bis 48 Stunden lang mit Wasserstoff zu regenerieren, wobei es vorteilhaft ist den Katalysator vor der eigentlichen Regenerierung mit dem im System enthaltenen Lösungsmittel, insbesondere mit Ammoniak, zu waschen.

In der WO 2008/104553 wird offenbart, dass Katalysatoren, die für die Hydrierung von TETA bzw. DETA eingesetzt werden, regeneriert werden können. Zur Regenerierung soll ein Verfahren gemäß der WO 99/33561 angewendet werden.

Die WO 99/33561 offenbart ein Verfahren zur Regenerierung von Raney-Katalysatoren, wobei zunächst die Abtrennung der Katalysatoren vom Reaktionsmedium erfolgt und der abgetrennte Katalysator mit einer wässrigen basischen Lösung behandelt wird, die eine Konzentration an basischen Ionen von mehr als 0,01 mol/kg aufweist und das Gemisch bei Temperaturen von weniger als 130°C 1 bis 10 Stunden ggf. in Gegenwart von Wasserstoff hält. Anschließend wird der Katalysator mit Wasser oder einer basischen Lösung gewaschen, bis das Waschwasser einen pH-Wert im Bereich von 12 bis 13 aufweist.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert.

Beim Suspensionsverfahren wird ebenfalls bevorzugt in situ regeneriert.

Dabei wird im Allgemeinen der gesamte Katalysator regeneriert.

Die Regenerierung erfolgt üblicherweise während einer kurzzeitigen Abstellung.

Regenerierung mit flüssigem Ammoniak und Wasserstoff

In einer besonders bevorzugten Ausführungsform werden Raney-Katalysatoren regeneriert, in dem die Raney-Katalysatoren mit flüssigem Ammoniak und Wasserstoff behandelt werden. Dabei sollte die Regenerierung mit einfachen technischen Mitteln ermöglicht werden. Zudem sollte die Regenerierung mit möglichst wenig Zeitaufwand erfolgen, um Stillzeiten in Folge der Katalysator-Regenerierung zu verringern. Weiterhin sollte die Regenerierung die weitestgehend vollständige Wiederherstellung der Aktivität der eingesetzten Katalysatoren ermöglichen.

Demgemäß betrifft diese besonders bevorzugte Ausführungsform ein

Verfahren zur Regenerierung von Raney-Katalysatoren, die bei der Umsetzung von EDDN bzw. EDMN mit Wasserstoff eingesetzt werden, in dem man den Katalysator mit flüssigem Ammoniak mit einem Wassergehalt von weniger als 5 Gew.% und Wasserstoff mit einem Partialdruck von 0,1 bis 40 MPa im Temperaturbereich von 50 bis 200 °C mindestens 0,1 Stunden behandelt.

In dieser bevorzugten Ausführungsform werden die zu vor beschriebenen dotierten und undotierten Raney-

Katalysatoren regeneriert.
Es werden besonders bevorzugt solche Raney-Katalysatoren eingesetzt, die bei der Umsetzung von EDDN bzw. EDMN mit Wasserstoff eingesetzt werden.
Insbesondere bevorzugt wird mittels dieser bevorzugten Ausführungsform Raney-Co regeneriert.
Zur Regenerierung wird der Raney-Katalysator mit Ammoniak behandelt.
Der eingesetzte Ammoniak enthält in dieser besonders bevorzugten Ausführungsform weniger als 5 Gew.-%, bevorzugt weniger als 3 Gew.-% und ganz besonders bevorzugt weniger als 1 Gew.-% Wasser. Solch "wasserfreier" Ammoniak ist ein im Handel allgemein erhältliches Produkt.
Die Regenerierung kann in allen Reaktoren erfolgen, die für die Hydrierung von EDDN bzw. EDMN zu TETA bzw. DETA eingesetzt werden können, und die nachfolgend und vorangehend beschrieben sind. Beispielsweise kann die Hydrierung in einem Rührreaktor, Strahlschlaufenreaktor, Strahldüsenreaktor, Blasensäulenreaktor, Rohrreaktor aber auch Rohbündelreaktor bzw. in einer Kaskade derartiger gleicher oder verschiedener Reaktoren durchgeführt werden. Die Hydrierung kann kontinuierlich oder diskontinuierlich erfolgen.
Bei der diskontinuierlichen Fahrweise wird der vorzugsweise Reaktor vor der Behandlung mit Ammoniak dazu zunächst geleert, beispielsweise in dem man den Reaktorinhalt aus dem Reaktor entfernt, z.B. durch Pumpen oder Ablassen. Die Leerung des Reaktors sollte weitestgehend vollständig sein. Bevorzugt sollten mehr als 80 Gew.-%, besonders bevorzugt mehr als 90 Gew.-% und ganz besonders bevorzugt mehr als 95 Gew.-% des Reaktorinhalts abgelassen oder abgepumpt werden.
Bei der kontinuierlichen Fahrweise wird bevorzugt die Zufuhr an Edukten unterbrochen und stattdessen flüssiger Ammoniak zugeführt.
Bei der kontinuierlichen Fahrweise kann der flüssige Ammoniak auch durch Kondensationsreaktionen innerhalb des Reaktors stammen, beispielsweise aus der Kondensation von EDA zu AEPIP.
Die Behandlung des Katalysators mit flüssigem Ammoniak erfolgt in dieser besonders bevorzugten Ausführungsform bei einer Temperatur von 50 bis 350°C, bevorzugt 150 bis 300°C, besonders bevorzugt 200 bis 250°C.
Die Dauer der Behandlung beträgt bevorzugt 0,1 bis 100 Stunden, bevorzugt 0,1 bis 10 Stunden und insbesondere bevorzugt 0,5 bis 5 Stunden.
Das Gewichtsverhältnis an zugeführter Menge Ammoniak zu Katalysator liegt bevorzugt im Bereich von 1 : 1 bis 1000 : 1, besonders bevorzugt im Bereich von 50 : 1 bis 200 : 1.
Es ist weiterhin bevorzugt, dass während der Behandlung mit Ammoniak der Ammoniak zirkuliert wird, beispielsweise durch Umpumpen oder bevorzugt durch Rühren.
Die Behandlung des Katalysators mit Ammoniak findet in der besonders bevorzugten Ausführungsform in Gegenwart von Wasserstoff statt. Der Wasserstoffpartialdruck bei der Behandlung mit Ammoniak liegt bevorzugt im Bereich von 1 bis 400 bar, besonders bevorzugt bei 5 bis 300 bar.
In einer besonders bevorzugten Ausführungsform beträgt die Konzentration an Anionen im flüssigem Ammoniak weniger als 0,01 mol/kg , ganz besonders bevorzugt weniger als 0,0099 mol/kg und insbesondere bevorzugt weniger 0,005 mol/kg.
Nach der Behandlung mit Ammoniak, kann Ammoniak vom Katalysator abgetrennt wird. Dies erfolgt beispielsweise durch Entleerung des Reaktors und/oder durch Abstellen der Ammoniak-Zufuhr.
Vor- und nach der Behandlung des Raney-Katalysators mit flüssigem Ammoniak kann der Raney-Katalysator ein oder mehrere Male mit organischem Lösungsmittel und/oder Wasser gespült werden.
Die Behandlung des Katalysators mit organischem Lösungsmittel und/oder Wasser nach der Abtrennung von Ammoniak bzw. nach Abstellung der Ammoniak-Zuführung ist jedoch nicht zwingend erforderlich, da das Ammoniak im Verlaufe der nachfolgenden Hydrierung nicht stört und kontinuierlich aus dem Reaktor ausgetragen werden kann.

Reaktionsbedingungen bei der Hydrierung

Die Herstellung von TETA bzw. DETA erfolgt im Allgemeinen durch Umsetzung von EDDN bzw. EDMN mit Wasserstoff in Gegenwart eines Hydrierkatalysators und eines organischen Lösungsmittels.
Die Temperaturen liegen im Allgemeinen in einem Bereich von 60 bis 150°C, bevorzugt von 80 bis 140°C, insbesondere bei 100 bis 130°C.
Der bei der Hydrierung herrschende Druck liegt im Allgemeinen bei 5 bis 400 bar, bevorzugt bei 60 bis 325 bar, besonders bevorzugt bei 100 bis 280 bar und insbesondere bevorzugt bei 170 bis 240 bar.
In einer besonders bevorzugten Ausführungsform liegt der Druck bei der Hydrierung bei Verwendung von Raney-Katalysatoren im Bereich von 170 bis 240 bar, da in diesem Druckbereich die Bildung von AEPIP verringert werden kann. Die Bildung von AEPIP kann die Deaktivierung des Katalysators beschleunigen.
Demgemäß betrifft die besonders bevorzugte Ausführungsform die
Herstellung von TETA und/oder DETA durch Umsetzung von EDDN und/oder EDMN mit Wasserstoff in Gegenwart eines Katalysators, dadurch gekennzeichnet, dass als Katalysator ein Katalysator von Raney-Typ eingesetzt wird und

der Druck bei Hydrierung im Bereich von 170 bis 240 bar liegt.

In einer bevorzugten Ausführungsform wird EDDN beziehungsweise das Aminonitrilgemisch enthaltend EDDN mit einer Rate der Hydrierung zugeführt, die nicht größer ist als die Rate, mit der EDDN und gegebenenfalls die übrigen Komponenten des Aminonitrilgemisches mit Wasserstoff bei der Hydrierung reagieren.

Bei der Hydrierung von EDDN zu TETA werden im Allgemeinen mindestens vier Mole Wasserstoff pro Mol EDDN benötigt.

Bei der Hydrierung von EDMN zu DETA werden im Allgemeinen mindestens zwei Mole Wasserstoff pro Mol EDMN benötigt.

Reaktor

Die Umsetzung von EDDN bzw. EDMN mit Wasserstoff in Gegenwart von Katalysatoren kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des EDDN bzw. EDMN und des Katalysators mit dem Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden.

Die Hydrierung an einem Festbettkatalysator findet bevorzugt in einem oder mehreren Rohrreaktoren aber auch Rohrbündelreaktoren statt.

Die Hydrierung der Nitrilgruppen findet unter Freisetzung von Wärme statt, die in der Regel abgeführt werden muss. Die Wärmeabfuhr kann durch eingebaute Wärmeüberträgerflächen,

Kühlmäntel oder Außen liegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Insbesondere kann der Kreislaufstrom mittels eines externen Wärmeüberträgers auf einfache und kostengünstige Weise gekühlt und somit die Reaktionswärme abgeführt werden.

Der Reaktor kann auch adiabat betrieben werden. Bei adiabatem Betrieb des Reaktors kann der Temperaturanstieg im Reaktionsgemisch durch Abkühlung der Zuläufe oder durch Zufuhr von "kaltem" organischem Lösungsmittel begrenzt werden.

Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Anordnung des Katalysators

Der Katalysator kann in einem Festbett angeordnet (Festbettfahrweise) sein oder im Reaktionsgemisch suspendiert (Suspensionsfahrweise) sein.

Suspensionsfahrweise

In einer besonders bevorzugten Ausführungsform ist der Katalysator im zu hydrierenden Reaktionsgemisch suspendiert.

Die Absetzgeschwindigkeit des Hydrierkatalysators in dem gewählten Lösungsmittel sollte niedrig sein, damit der Katalysator gut in Suspension gehalten werden kann.

Die Partikelgröße der eingesetzten Katalysatoren beträgt bei der Suspensionsfahrweise daher bevorzugt zwischen 0,1 und 500 $\mu$m, insbesondere 1 und 100 $\mu$m.

Wird die Hydrierung von EDDN bzw. EDMN in Suspensionsfahrweise kontinuierlich durchgeführt, so werden EDDN bzw. EDMN bevorzugt kontinuierlich dem Reaktor zugeführt und aus dem Reaktor kontinuierlich ein Strom entfernt, der die Hydrierungsprodukte TETA bzw. DETA enthält.

Bei der diskontinuierlichen Suspensionsfahrweise werden EDDN bzw. EDMN, ggf. zusammen mit organischem Lösungsmittel vorgelegt.

Die Menge an Katalysator bei der diskontinuierlichen Suspensionsfahrweise beträgt bevorzugt 1 bis 60 Gew.-%, besonders bevorzugt 5 bis 40 Gew.-%, und ganz besonders bevorzugt 20 bis 30 Gew.-%, bezogen auf das gesamte Reaktionsgemisch.

Die Verweilzeit im Reaktor beträgt bei diskontinuierlicher Suspensions-Fahrweise bevorzugt 0,1 bis 6 Stunden,

besonders bevorzugt 0,5 bis 2 Stunden.

Die Verweilzeit im Reaktor beträgt bei kontinuierlicher Suspensions-Fahrweise bevorzugt 0,1 bis 6 Stunden, besonders bevorzugt 0,5 bis 2 Stunden.

Die Katalysatorbelastung beträgt bei der kontinuierlichen Suspensionsfahrweise bevorzugt 0,1 bis 10 kg, bevorzugt 0,5 bis 5 kg EDDN + EDMN pro kg-Katalysator und Stunde.

In einer besonders bevorzugten Ausführungsform beträgt die Katalysatorbelastung, bezogen auf die Katalysator-Oberfläche bevorzugt $10^{-6}$ bis $10^{-4}$ kg EDDN+EDMN pro $m^2$ Katalysatoroberfläche und Stunde, wobei die Katalysatoroberfläche gemäß der BET-Methode bestimmt wird (DIN 66131). Besonders bevorzugt beträgt die Katalysatorbelastung, bezogen auf die Katalysator-Oberfläche $0{,}25 \cdot 10^{-5}$ bis $5 \cdot 10^{-5}$ kg EDDN+EDMN pro $m^2$ Katalysatoroberfläche und Stunde und ganz besonders bevorzugt $0{,}5 \cdot 10^{-5}$ bis $2 \cdot 10^{-5}$ kg EDDN+EDMN pro $m^2$ Katalysatoroberfläche und Stunde.

Demgemäß betrifft die besonders bevorzugte Ausführungsform die

Herstellung von TETA und/oder DETA durch Umsetzung von EDDN und/oder EDMN mit Wasserstoff in Gegenwart eines Katalysators im Suspension, dadurch gekennzeichnet, dass die Katalysatorbelastung, bezogen auf die Katalysator-Oberfläche $10^{-6}$ bis $10^{-4}$ kg EDDN+EDMN pro $m^2$ Katalysatoroberfläche und Stunde beträgt, wobei die Katalysatoroberfläche gemäß der BET-Methode bestimmt wird.

Wird die Reaktion in Suspensionsfahrweise in einem Rührreaktor durchgeführt, so liegt der Leistungseintrag über den Rührer bevorzugt bei 0,1 bis 100 KW pro $m^3$.

Gebrauchter Katalysator kann durch Filtration, Zentrifugieren oder Querstromfiltration abgetrennt werden. Dabei kann es notwendig sein, Verluste an ursprünglicher Katalysatormenge durch Abrieb und/oder Desaktivierung durch Zugabe von frischem Katalysator auszugleichen.

Festbettfahrweise

In einer weiteren, weniger bevorzugten Ausführungsform ist der Katalysator in einem Katalysatorfestbett angeordnet.

Die Katalysatorbelastung bei der kontinuierlichen Hydrierung im Festbettreaktor, beispielsweise einem Rohrreaktor oder Rohrbündelreaktor, beträgt bevorzugt 0,1 bis 10 kg, bevorzugt 0,5 bis 5 kg EDDN + EDMN pro kg Katalysator und Stunde.

In einer besonders bevorzugten Ausführungsform beträgt die Katalysatorbelastung, bezogen auf die Katalysator-Oberfläche bevorzugt $10^{-6}$ bis $10^{-4}$ kg EDDN+EDMN pro $m^2$ Katalysatoroberfläche und Stunde, wobei die Katalysatoroberfläche gemäß der BET-Methode bestimmt wird (DIN 66131). Besonders bevorzugt beträgt die Katalysatorbelastung, bezogen auf die Katalysator-Oberfläche $0{,}25 \cdot 10^{-5}$ bis $5 \cdot 10^{-5}$ kg EDDN+EDMN pro $m^2$ Katalysatoroberfläche und Stunde und ganz besonders bevorzugt $0{,}5 \cdot 10^{-5}$ bis $2 \cdot 10^{-5}$ kg EDDN+EDMN pro $m^2$ Katalysatoroberfläche und Stunde.

Demgemäß betrifft die besonders bevorzugte Ausführungsform die

Herstellung von TETA und/oder DETA durch Umsetzung von EDDN und/oder EDMN mit Wasserstoff in Gegenwart eines Katalysators im Festbett, dadurch gekennzeichnet, dass die Katalysatorbelastung, bezogen auf die Katalysator-Oberfläche $10^{-6}$ bis $10^{-4}$ kg EDDN+EDMN pro $m^2$ Katalysatoroberfläche und Stunde beträgt, wobei die Katalysatoroberfläche gemäß der BET-Methode bestimmt wird.

Im Fall eines Festbettkatalysators wird dieser im Allgemeinen in Sumpf- oder Rieselfahrweise mit EDDN bzw. EDMN beaufschlagt.

Reaktionsaustrag

Der Reaktionsaustrag aus der Hydrierung enthält in der Regel auch weitere höher- bzw. niedriger siedende organische Substanzen als Nebenprodukte, wie Methylamin, AEPIP, PIP oder TEPA oder basische Verbindungen bzw. Additive, die vor oder während der Hydrierung zugeführt wurden, beispielsweise Alkalimetallhydroxide, Alkoholate, Amide, Amine und Ammoniak. Der Hydrieraustrag enthält weiterhin bevorzugt organisches Lösungsmittel, welches während der Hydrierung zugegen war, vorzugsweise das organische Lösungsmittel, welches auch bei der Behandlung mit Adsorbens zugegen war, insbesondere THF.

Der Reaktionsaustrag enthält weiterhin bevorzugt Destillationsmittel, insbesondere Toluol, welches bevorzugt bei der destillativen Abreicherung von Wasser nach der EDDN- bzw. EDMN-Herstellung eingesetzt wurde.

Der Reaktionsaustrag enthält im Allgemeinen auch noch geringe Wassermengen.

In der Regel entsprechen die Wassermengen, die im Austrag aus der Hydrierung enthalten sind, den Mengen, die aus der EDDN- bzw. EDMN-Herstellung und der bevorzugt erfolgenden Aufarbeitung stammen.

Aufreinigung (Allgemein)

Im Anschluss an die Hydrierung kann der Austrag aus der Hydrierung gegebenenfalls weiter aufgereinigt

werden.
Der Katalysator kann nach dem Fachmann bekannten Methoden abgetrennt werden.
In der Regel wird nach Abtrennung des Katalysators der während der Hydrierung vorhandene Wasserstoff abgetrennt.

Abtrennung von Wasserstoff

Die Abtrennung von Wasserstoff erfolgt bevorzugt durch Absenkung des Drucks, bei dem die Hydrierung durchgeführt wurde, auf einen Wert, bei dem Wasserstoff gasförmig ist, die anderen Komponenten im Reaktionsaustrag aber in der Flüssigphase vorliegen. Vorzugsweise wird der Reaktionsaustrag von einem Hydrierdruck von bevorzugt 60 bis 325 bar, besonders bevorzugt 100 bis 280 bar, und ganz besonders bevorzugt 170 bis 240 bar auf einen Druck von 5 bis 50 bar in einen Behälter entspannt. Am Kopf des Behälters werden Wasserstoff und ggf. Ammoniak, sowie geringe Menge an verdampften Leichtsiedern, wie THF, erhalten. Wasserstoff und ggf. Ammoniak können in die Hydrierung von EDDN bzw. EDMN zurückgeführt werden. Beispielsweise kann THF auskondensiert und zurück gewonnen werden. Alternativ kann THF durch Abgaswäsche mit einem höher siedenden Lösungsmittel, wie beispielsweise Toluol oder TETA, zurück gewonnen werden.

Abtrennung der organischen Lösungsmittel

Im Reaktionsaustrag vorhandene organische Lösungsmittel werden im Allgemeinen ebenfalls destillativ abgetrennt.
Insbesondere können die Hauptprodukte (TETA bzw. DETA) gemeinsam oder einzeln nach dem Fachmann bekannten Methoden aus dem Reaktionsprodukt isoliert werden. Sofern die beiden Hauptprodukte gemeinsam isoliert werden, beispielsweise durch eine Destillation, können sie anschließend in die beiden Einzelprodukte getrennt werden. Letztendlich erhält man somit reines TETA und reines DETA. Sonstige Verunreinigungen, Nebenprodukte oder weitere Ethylenamine wie TEPA oder PIP können ebenfalls mit dem Fachmann bekannten Methoden aus dem jeweiligen Produkt abgetrennt werden. Gegebenenfalls kann TETA auch gemeinsam mit in geringen Mengen gebildeten Diaminoethylpiperazin oder Piperazinyl-ethyl-ethylendiamin isoliert werden.
Die Aufarbeitung der Hydrierausträge aus der Hydrierung von EDDN erfolgt bevorzugt durch Destillation.

THF-Abtrennung

Wenn der Hydrieraustrag THF enthält, dann ist es bevorzugt, das THF in das Verfahren zurückzuführen. Insbesondere ist es bevorzugt, das THF, welches bei der Hydrierung zugegen war, wieder zur Behandlung von EDDN und/oder EDMN mit Adsorbens einzusetzen.
Hierbei ist es allerdings erforderlich, dass das THF nahezu wasserfrei zurückgeführt wird, da gefunden wurde, dass geringe Mengen Wasser bei der Behandlung mit Adsorbens das Absorptionsvermögen des Adsorbens verringern können und bei der Hydrierung von EDDN bzw. EDMN polare Verunreinigungen eingeschleppt werden können, die zu unerwünschten Nebenreaktionen führen. THF und Wasser bilden allerdings ein leichtsiedendes Azeotrop.
Enthält der Hydrieraustrag THF, so kann die Abtrennung von Wasser und THF beispielsweise als 2 Druck-Destillation erfolgen

In einer besonders bevorzugten Ausführungsform erfolgt die Abtrennung von THF durch die Auftrennung eines Reaktionsaustrags, der bei der Umsetzung von EDDN bzw. EDMN mit Wasserstoff in Gegenwart von THF und eines Katalysators anfällt, welcher TETA bzw. DETA, Wasser sowie ggf. höher und niedriger als TETA bzw. DETA siedende organische Verbindungen enthält, dadurch gekennzeichnet, dass man

i) den Reaktionsaustrag nach Abtrennung von Wasserstoff einer Destillationskolonne DK1 zuführt, in der ein THF/Wasser-Azeotrop über Kopf abgetrennt wird, welches ggf. noch weitere organische Verbindungen mit einem niedrigeren Siedepunkt als TETA bzw. DETA enthält, und in der ein Sumpfprodukt abgetrennt wird, welches TETA bzw. DETA enthält, und
ii) das Sumpfprodukt aus Stufe i) in eine Destillationskolonne DK2 leitet und THF über Kopf abtrennt und am Sumpf der Kolonne einen Strom abzieht, der TETA bzw. DETA enthält, und
iii) den am Kopf der Kolonne DK1 abgezogenen Strom aus Stufe i) kondensiert und dem Kondensat oder einem Teil des Kondensats ein organisches Lösungsmittel, das im Wesentlichen nicht mit Wasser mischbar ist, in einer solchen Menge zuführt, dass ein Phasenzerfall auftritt und das so erhaltene Gemisch in einem Phasenscheider trennt, wobei die sich bildende organische Phase, enthaltend THF und das organische Lösungsmittel, welches im Wesentlichen nicht mit Wasser mischbar ist, in die Kolonne DK1 zurückführt und die Wasserphase ausschleust.

In der besonders bevorzugten Ausführungsform wird zunächst Wasserstoff vom Reaktionsaustrag abgetrennt. Die Abtrennung von Wasserstoff erfolgt, wie voranstehend beschrieben, bevorzugt durch Absenkung des Drucks bei dem die Hydrierung durchgeführt wurde auf einen Druck, bei dem Wasserstoff gasförmig ist, die anderen Komponenten

im Reaktionsaustrag aber in der Flüssigphase vorliegen. Vorzugsweise wird der Reaktionsaustrag von einem Hydrierdruck von bevorzugt 60 bis 325 bar, besonders bevorzugt 100 bis 280 bar, und ganz besonders bevorzugt 170 bis 240 bar auf einen Druck von 5 bis 50 bar in einen Behälter entspannt. Am Kopf des Behälters werden Wasserstoff und ggf. Ammoniak, sowie geringe Menge an verdampften Leichtsiedern, wie THF, erhalten. Wasserstoff und ggf. Ammoniak können in die Hydrierung von EDDN bzw. EDMN zurückgeführt werden. THF kann auskondensiert werden und zurückgewonnen werden. Alternativ kann THF durch Abgaswäsche mit einem höhersiedenden Lösungsmittel wie beispielsweise Toluol oder TETA zurück gewonnen werden.

In der besonders bevorzugten Ausführungsform wird nach Abtrennung von Wasserstoff der Reaktionsaustrag einer Kolonne DK1 zugeführt.

Hierzu wird bevorzugt der nach der Entspannung flüssig gebliebene Anteil des Reaktionsaustrags in eine Kolonne DK1 geleitet.

Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Die Kolonne ist bevorzugt als Bodenkolonne ausgestaltet.

Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.

Die Kolonne weist bevorzugt einen Abtriebs- und einen Verstärkungsteil. Sie kann aber auch nur einen Abtriebsteil aufweisen.

Die Anzahl der theoretischen Böden liegt im Allgemeinen im Bereich von 5 bis 30, vorzugsweise 10 bis 20.

Der Druck der Kolonne wird vorzugsweise so gewählt wird, dass sich eine Sumpftemperatur im Bereich von 100 bis 250°C einstellt.

Vorzugsweise beträgt der Kopfdruck 1 bis 30 bar, besonders bevorzugt 3 bis 25 bar.

In der Regel liegt die Betriebstemperatur des Kondensators im Bereich von 30 bis 70°C, vorzugsweise 35 bis 50°C.

In Allgemeinen werden Leichtsieder, wie Ammoniak oder Methylamin, nicht kondensiert und als gasförmiger Strom ausgeschleust. Dieser Strom kann nachfolgend einer Verbrennung zugeführt werden.

Im Kondensator fällt als Kondensat überwiegend das abgetrennte Azeotrop aus Wasser und THF an.

In der besonders bevorzugten Ausführungsform wird dem Kondensat oder einem Teil des Kondensats ein organisches Lösungsmittel zugeführt, welches im Wesentlichen nicht mit Wasser mischbar ist und welches unter den Destillationsbedingungen in der Kolonne DK1 einen höheren Siedepunkt hat, als das sich bildende THF/Wasser-Azeotrop, das am Kopf der Kolonne abgezogen wird.

Als organische Lösungsmittel, die im Wesentlichen nicht mit Wasser mischbar sind, werden im Rahmen der vorliegenden Erfindung solche organischen Lösungsmittel verstanden, in denen weniger als 500 Gew.-ppm Wasser gelöst werden können.

Bevorzugte organische Lösungsmittel, die im Wesentlichen nicht mit Wasser mischbar sind, sind Toluol, n-Heptan, n-Octan, n-Nonan und ähnliche.

Besonders bevorzugt werden solche organischen Lösungsmittel, die im Wesentlichen nicht mit Wasser mischbar sind, eingesetzt, die auch bevorzugte Lösungsmittel bei der EDDN-bzw. EDMN-Herstellung sind.

Ganz besonders bevorzugt wird Toluol eingesetzt, da dieses bereits bevorzugt bei der EDDN-bzw. EDMN-Herstellung eingesetzt wird.

Die Menge an zugeführtem organischem Lösungsmittel, das im Wesentlichen nicht mit Wasser mischbar ist, wird im Allgemeinen so gewählt, dass Phasenzerfall auftritt und die Phasen mittels der üblichen technischen Maßnahmen, wie Trennung in einem Phasentrenngefäß, getrennt werden können.

Das Gewichtsverhältnis von zugeführtem organischem Lösungsmittel, das im Wesentlichen nicht mit Wasser mischbar ist, zu Kondensat beträgt bevorzugt 0.1 : 1 bis 10 : 1, besonders bevorzugt 0,5 : 1 bis 5 : 1 und ganz besonders bevorzugt 0,8 : 1 bis 2 : 1.

Das so erhaltene Gemisch von Kondensat und organischem Lösungsmittel, das im Wesentlichen nicht mit Wasser mischbar, wird bevorzugt in einen Phasenscheider geleitet, wo es in eine wässrige Phase und eine Phase, enthaltend THF und das im wesentlichen nicht mit Wasser mischbare Lösungsmittel, zerfällt.

Vorzugsweise wird die Phase, die THF und das im Wesentlichen nicht mit Wasser mischbare Lösungsmittel enthält, in den oberen Bereich der Kolonne DK1, vorzugsweise auf den Kopf der Kolonne DK1, zurückgeführt.

Bevorzugt wird die gesamte Phase, die THF und das organische Lösungsmittel, das im Wesentlichen nicht mit Wasser mischbar ist, enthält, in den oberen Bereich der Kolonne DK1 zurückgeführt.

Durch die Abtrennung des Wassers am Kopf der Kolonne mittels Zugabe eines organischen Lösungsmittels, das im Wesentlichen nicht mit Wasser mischbar ist, und anschließender Phasentrennung, ist es möglich einen Sumpfaustrag zu erhalten, der nur geringe Mengen Wasser enthält. Vorzugsweise enthält der Sumpfaustrag weniger als 1 Gew.-%, besonders bevorzugt weniger als 1000 Gew.-ppm und besonders bevorzugt weniger als 200 Gew.-ppm Wasser.

Der Sumpfaustrag aus Kolonne DK1 enthält weiterhin TETA bzw. DETA, THF, das im wesentlichen nicht mit Wasser mischbare Lösungsmittel, und ggf. weiteres organisches Lösungsmittel (welches aus der Entwässerung und Phasentrennung stammt) sowie im Allgemeinen organische Nebenprodukte, wie PIP, AEPIP und TEPA.

In der besonders bevorzugten Ausführungsform wird das Sumpfprodukt aus Kolonne DK1 in eine Destillationskolonne DK2 geleitet, in der THF über Kopf abgetrennt wird und am Sumpf der Kolonne ein Strom abgezogen wird, der TETA bzw. DETA sowie das im Wesentlichen nicht mit Wasser mischbare Lösungsmittel und ggf. zusätzliches Toluol enthält.

Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Die Kolonne ist bevorzugt als Bodenkolonne ausgestaltet.

Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.

Die Kolonne weist bevorzugt nur einen Abtriebsteil auf.

Die Anzahl der theoretischen Böden liegt im Allgemeinen im Bereich von 5 bis 30, vorzugsweise 10 bis 20.

Der Kopfdruck beträgt besonders bevorzugt 200 mbar bis 5 bar, besonders bevorzugt 500 mbar bis 2 bar.

Im Kolonnensumpf wird bevorzugt eine Temperatur eingestellt, die oberhalb der Verdampfungstemperatur von THF liegt, so dass THF im Wesentlichen vollständig in die Gasphase übergeht. Besonders bevorzugt wird eine Temperatur am Kolonnensumpf eingestellt, die im Bereich von 100 bis 250°C liegt.

Der Kondensator der Destillationskolonne DK2 wird in der Regel bei einer Temperatur betrieben, bei der der überwiegende Teil des THF bei dem entsprechenden Kopfdruck kondensiert wird. In der Regel liegt die Betriebstemperatur des Kondensators im Bereich von 30 bis 70°C, vorzugsweise 35 bis 50°C.

Im Kondensator fällt ein Kondensat an, welches im wesentlichen THF enthält. Bevorzugt enthält dieses THF weniger als 200 Gew.-ppm, besonders bevorzugt weniger als 100 Gew.-ppm Wasser, so dass es besonders geeignet ist für eine Rückführung in die Aufarbeitung des Reaktionsaustrags oder der EDDN bzw. EDMN-Herstellung. So kann zwischen der EDDN bzw. EDMN-Hydrierung und der EDDN- bzw. EDMN-Herstellung ein Verbund geschaffen werden, der die Mengen an benötigten organischen Lösungsmitteln reduziert.

Ggf. kann das Kondensat am Kopf der Kolonne DK2 neben THF auch noch Spuren von dem organischen Lösungsmittel, welches im Wesentlichen nicht mit Wasser mischbar ist, enthalten. Das Kondensat kann aber trotzdem, wie zuvor beschrieben, in die EDDN- bzw. EDMN-Aufarbeitung zurückgeführt werden, da diese Lösungsmittel, wie voranstehend beschrieben, ebenfalls ein bevorzugtes organisches Lösungsmittel in dieser Stufe sind. Bevorzugt wird jedoch die Menge an organischem Lösungsmittel, welches im Wesentlichen nicht mit Wasser mischbar ist, im Kondensat verringert, indem am Kopf der Kolonne ein Vorkondensator vorgeschaltet ist, der im Temperaturbereich von 80 bis 150°C, vorzugsweise 100 bis 130°C betrieben wird. Alternativ können die Anzahl der Böden im Verstärkungsteil der Kolonne DK2 erhöht werden und/oder ein Teil des Kondensat als Rücklauf auf die Kolonne gegeben werden. Es ist allerdings auch möglich den Anteil an organischem Lösungsmittel, welches im Wesentlichen nicht mit Wasser mischbar ist, im Kopfdestillat zu verringern, in dem der Zulauf zur Kolonne DK2 gekühlt wird und/oder die Sumpftemperatur in der Kolonne DK2 so eingestellt wird, dass nur geringe Menge des mit Wasser nicht mischbaren organischen Lösungsmittels in die Gasphase überführt werden.

Am Sumpf der Kolonne DK2 fällt in der Regel ein Sumpfprodukt an, welches TETA bzw. DETA, Toluol, sowie im Allgemeinen die Nebenprodukte AEPIP, PIP und TEPA enthält.

In einer weiteren besonders bevorzugten Ausführungsform wird THF, welches durch 2-Druck-Destillation oder welches gemäß der besonders bevorzugten Ausführungsform am Kopf der

Kolonne DK 2 erhalten wird, vor der Rückführung in das Verfahren, insbesondere vor der Rückführung in die Adsorber-Stufe mit einem Molsieb weiter entwässert. Bevorzugt weist das Molsieb einen Porendurchmesser von kleiner als 4 A auf, so dass nur Wasser und Ammoniak zurückgehalten werden, andere Amine wie Methylamin und Ethylamin aber nicht. Die Aufnahmekapazität des Molsiebs als Adsorbens für die Abtrennung von Wasser wird dadurch erhöht.

Aufarbeitung des Sumpfprodukts

Dieser Sumpfaustrag kann nach herkömmlichen Methoden weiter aufgearbeitet werden und in die Einzelbestandteile aufgetrennt werden.

In einer bevorzugten Ausführungsform wird das Sumpfprodukt aus Kolonne DK2 in eine Kolonne DK3 geleitet, in der am Kopf ein Strom abgezogen wird, der überwiegend Toluol und/oder das im Wesentlichen nicht mit Wasser mischbare Lösungsmittel enthält, und als Sumpfprodukt ein Strom abgezogen wird, der überwiegend TETA bzw. DETA, AEPIP sowie im Allgemeinen die Nebenprodukte PIP, AEPIP sowie TEPA enthält.

Die genauen Betriebsbedingungen der Destillationskolonne können entsprechend der Trennleistung der verwendeten

Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.
Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Betten vorliegen.
Die Kolonne weist bevorzugt einen Abtriebs- und einen Verstärkungsteil auf.
Der Sumpfaustrag aus Kolonne DK2 wird vorzugsweise in einem räumlichen Bereich zwischen 30% und 90% der theoretischen Böden der Destillationskolonne zugeführt (von unten gezählt), besonders bevorzugt in einem räumlichen Bereich zwischen 50% und 80% der theoretischen Böden der Destillationskolonne. Beispielsweise kann die Zuführung etwas oberhalb der Mitte der theoretischen Böden erfolgen. Die optimale Zulaufstelle kann vom Fachmann mit den üblichen Berechnungswerkzeugen ermittelt werden.
Die Anzahl der theoretischen Böden liegt im Allgemeinen im Bereich von 3 bis 25, vorzugsweise 5 bis 15.
Besonders bevorzugt wird eine Temperatur am Kolonnensumpf eingestellt, die im Bereich von 100 bis 250°C liegt.
Der Kopfdruck beträgt bevorzugt 10mbar bis 1 bar, besonders bevorzugt 30 mbar bis 500 mbar. Der Kondensator der Destillationskolonne wird in der Regel bei einer Temperatur betrieben, bei der der überwiegende Teil des Toluols und/oder des im Wesentlichen nicht mit Wasser mischbaren Lösungsmittels bei dem entsprechenden Kopfdruck kondensiert wird.
In der Regel liegt die Betriebstemperatur des Kondensators im Bereich von 30 bis 70°C, vorzugsweise 35 bis 50°C.
Im Kondensator fällt ein Kondensat an, welches im wesentlichen Toluol und/oder das im Wesentlichen nicht mit Wasser mischbare organische Lösungsmittel enthält. Das so erhaltene Toluol und/oder das im Wesentlichen nicht mit Wasser mischbare organische Lösungsmittel können in den Prozess zurückgeführt werden, beispielsweise in dem es dem Kondensat aus Kolonne DK1 zugeführt wird. Toluol und/oder das im Wesentlichen nicht mit Wasser mischbare organische Lösungsmittel kann aber auch der EDDN- bzw. EDMN-Aufarbeitung zugeführt werden, beispielsweise vor der Entspannungsverdampfung. Auf diese Weise ist es möglich einen wirtschaftlichen Verbund zu erzielen.
Am Sumpf der Kolonne DK3 fällt in der Regel ein Strom an, welcher TETA bzw. DETA, sowie im Allgemeinen die Nebenprodukte AEPIP, PIP und TEPA enthält.

Dieser Sumpfaustrag kann nach herkömmlichen Methoden weiter aufgearbeitet werden und in die Einzelbestandteile aufgetrennt werden.

In einer bevorzugten Ausführungsform wird der Sumpfaustrag aus Kolonne DK3 in eine Kolonne DK4 geleitet, in der am Kopf ein Gemisch aus PIP, AEPIP und DETA erhalten wird, am Sumpf ein Gemisch aus Pentaminen, wie TEPA und anderen Hochsiedern anfällt und als Seitenabzug ein TETA-Strom mit einer Reinheit von mehr als 99 Gew.-% abgezogen wird.
Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.
Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Betten vorliegen.
Die Kolonne weist bevorzugt einen Abtriebs- und einen Verstärkungsteil auf.
Der Sumpfaustrag aus Kolonne DK3 wird vorzugsweise in einem räumlichen Bereich zwischen 30% und 90% der theoretischen Böden der Destillationskolonne zugeführt (von unten gezählt), besonders bevorzugt in einem räumlichen Bereich zwischen 50% und 80% der theoretischen Böden der Destillationskolonne. Beispielsweise kann die Zuführung etwas oberhalb der Mitte der theoretischen Böden erfolgen. Die optimale Zulaufstelle kann vom Fachmann mit den üblichen Berechnungswerkzeugen ermittelt werden.
Die Anzahl der theoretischen Böden liegt im Allgemeinen im Bereich von 5 bis 30, vorzugsweise 10 bis 20.

Der Kopfdruck beträgt besonders bevorzugt 1 mbar bis 400 mbar, besonders bevorzugt 5 mbar bis 300 mbar. Im Kolonnensumpf wird bevorzugt eine Temperatur eingestellt, die oberhalb der Verdampfungstemperatur von Toluol liegt, so dass Toluol im Wesentlichen vollständig in die Gasphase übergeht.
Besonders bevorzugt wird eine Temperatur am Kolonnensumpf eingestellt, die im Bereich von 150 bis 250°C liegt.
Der Kondensator der Destillationskolonne wird in der Regel bei einer Temperatur von bevorzugt 30 bis 70°C, besonders bevorzugt 35 bis 50°C betrieben.
Im Kondensator fällt ein Kondensat an, welches im Wesentlichen ein Gemisch von DETA, PIP und AEPIP enthält.
Ein Teil des Kondensats kann als Rücklauf in die Kolonne DK4 zurückgeführt werden. Vorzugsweise werden 5 bis 40

Gew.-%, besonders bevorzugt 10 bis 25 Gew.-% des Kondensats als Rücklauf in die Kolonne DK4 zurückgeführt.
Am Sumpf der Kolonne DK4 fällt in der Regel ein Strom an, welcher im Wesentlichen en Gemisch aus Pentaaminen, wie TEPA, und anderen Hochsiedern enthält.
Als Seitenstrom wird TETA abgezogen. Der Seitenstrom wird vorzugsweise unterhalb der Zuleitung des Sumpfstroms aus Kolonne DK4 abgezogen, vorzugsweise im Bereich von 10% bis 60%, besonders bevorzugt im Bereich von 15 bis 35% der theoretischen Böden der Destillationskolonne (von unten gezählt). Der Seitenabzug enthält bevorzugt mehr als 99 Gew.-%, besonders bevorzugt mehr als 99,5 Gew.-% TETA.

Das durch das erfindungsgemäße Verfahren, sowie die bevorzugten Ausführungsformen hergestellte TETA bzw. DETA weist in der Regel eine hohe Qualität auf und ist somit besonders für weitere Umsetzungen geeignet, beispielsweise zur Umsetzung mit Epoxyverbindungen zur Herstellung von Epoxidharzen bzw. zur Umsetzung mit Säuren zur Herstellung von Amiden bzw. Polyamiden.
Ein weiterer Gegenstand der vorliegenden Erfindung ist deshalb auch die Herstellung von Epoxidharzen oder Amiden oder Polyamiden, dadurch gekennzeichnet, dass in einer ersten Stufe TETA und/oder DETA erfindungsgemäß hergestellt wird, und in einer zweiten Stufe das so erhaltene TETA bzw. DETA zu Epoxidharzen, Amiden oder Polyamiden umgesetzt wird.

Auf den beiliegenden Zeichnungen sind bevorzugte Ausführungsformen der Erfindung aufgeführt.

In Figur 1 ist die Herstellung von EDDN bzw. EDMN aus FA (1), EDA (2) und HCN (5) dargestellt, wobei zunächst FA (1) und EDA (2) zu EDFA und/oder EDMFA (4) umgesetzt werden, welches dann mit HCN (5) zu EDDN bzw. EDMN reagiert.
Die bevorzugten Verfahrensparameter können der voranstehenden Beschreibung entnommen werden. Zunächst wird FA (1) mit EDA (2) in den Kreislauf eines Schlaufenreaktors (I) eingemischt. In dem Schlaufenreaktor wird FA (1) mit EDA (2) zu EDFA und/oder EDMFA umgesetzt. Ein Teil des Reaktorinhalts des Schlaufenreaktors wird ausgeschleust (3) und in einen Rohrreakor (II) geleitet. Der Austrag (4) aus dem Rohrreaktor (II) wird am Eingang eines Rohrreaktors (III) an einer Einmischstelle mit HCN (5) und Toluol (6) vermischt und durch den Rohrreaktor (III) geleitet.
Am Ausgang des Rohrreaktors (III) wird das austretende Reaktionsgemisch (7) an einem Entspannungsventil entspannt. Die sich bildende gasförmige Phase (8), die überwiegend Wasser und Toluol enthält, wird an einem Kondensator (V) kondensiert. Nicht kondensierte Bestandteile (9), wie Ammoniak, HCN, Methanol oder $CO_2$, werden aus dem Verfahren ausgeschleust. Das am Kondensator (V) kondensierte Kondensat (10) wird in ein Phasentrenngefäß (VI) eingeleitet und in eine wässrige Phase (14) und eine Toluol-haltige Phase (11) getrennt.
Die wässrige Phase (14) aus dem Phasentrenngefäß (VI) kann in das Verfahren zurückgeführt werden, beispielsweise zur Herstellung einer wässrigen EDA-Lösung in Mischer (I) oder in eine biologische Abwasseraufbereitung eingeleitet werden (nicht gezeigt). Die wässrige Phase (14) kann auch in eine Kolonne K2 (VIII) eingeleitet werden, in der Wasser als Sumpfprodukt (16) von Leichtsiedern (15) abgetrennt wird. Die Leichtsieder (15), beispielsweise leichter als Wasser siedende Lösungsmittel oder leichtsiedende Wasser-Azeotrope oder HCN, können direkt auf den Kondensator (V) geführt werden. Nichtkondensierbare Bestandteile werden als Strom (9) aus dem Verfahren ausgeschleust.
Die Toluol-haltige Phase (11) kann als organisches Lösungsmittel in das Verfahren zurückgeführt werden und mit dem EDFA-haltigen Strom aus der EDFA-Herstellung vermischt werden. Gegebenenfalls können Verluste an Toluol durch eine Toluol-Ergänzung ergänzt werden. Die Toluol-haltige Phase (11) kann aber zusammen mit der flüssigen Phase (12) aus der FlashVorlage (IV) auch in eine Kolonne K1 (VII) eingeleitet werden.
Die bei der Entspannungsverdampfung flüssig gebliebene Phase (12) wird aus der FlashVorlage (Entspannungs-Behälter) (IV) und ebenfalls, ggf. zusammen mit der Toluol-haltigen Phase (11), auf den Kopf der Kolonne K1 (VII) geführt, um Wasser abzureichern.
In der Kolonne K1 (VII) wird ein gasförmiges, im Wesentlichen wässriges Kopfprodukt direkt auf den Kondensator (V) geführt und in das Phasen-Trenngefäß (VI) geleitet, wo die wässrige Phase (15), wie zuvor beschrieben ausgeschleust, in den Mischer (I) geleitet ,oder der Kolonne K2 (VIII) zugeführt werden kann.
Am Sumpf (17) der Kolonne K1 wird eine Mischung aus EDDN bzw.EDMN und Toluol erhalten. Die Mischung aus Toluol und EDDN bzw. EDMN (17) wird mit THF (18) verdünnt und in einem Adsorber (IX) mit Adsorbens, bevorzugt mit einem festen, sauren Adsorbens, behandelt. Aus dem Adsorber wird eine Mischung aus EDDN und/oder EDMN mit Toluol und THF erhalten, die nur noch geringe Mengen von Wasser enthält. Die EDDN- bzw. EDMN-Mischung kann in eine Hydrierung geleitet werden, in der EDDN bzw. EDMN zu TETA bzw. DETA hydriert wird.

In Figur 2 ist die Herstellung von TETA bzw. DETA aus EDDN bzw. EDMN dargestellt.
Die bevorzugten Verfahrensparameter können der voranstehenden Beschreibung entnommen werden.
EDDN bzw. EDMN, welches durch Umsetzung von EDFA und/oder EDMFA und HCN hergestellt werden kann, und welches aufgearbeitet wurde, vorzugsweise durch i) Abtrennung von Leichtsiedern, beispielsweise durch Strippen, Entspannungsverdampfung oder Destillation und ii) Abdestillation von Wasser, vorzugsweise in Gegenwart eines organischem Lösungsmittels, welches unter den Bedingungen der Wasserabtrennung einen Siedepunkt zwischen Wasser und EDDN bzw. EDMN aufweist oder welches ein mit Wasser leicht siedendes Azeotrop bildet, wird in der Figur 3 als "ungereinigtes" EDDN bezeichnet. Ein solch "ungereinigtes" EDDN bzw. EDMN wird mit THF (18) vermischt und in

einem Adsorber mit Adsorbens, bevorzugt festem, sauren Adsorbens, behandelt. Der Strom (1), der den Adsorber verlässt, wird in einen Hydrierreaktor (I) geleitet, in der das durch Adsorption "gereinigte" EDDN bzw. EDMN in Gegenwart von Wasserstoff (2) zu TETA bzw. DETA hydriert wird.

In Figur 3 ist die Herstellung von TETA bzw. DETA aus EDDN bzw. EDMN mit anschließender Aufarbeitung dargestellt.

Die bevorzugten Verfahrensparameter können der voranstehenden Beschreibung entnommen werden.

EDDN bzw. EDMN kann durch Umsetzung von EDFA und/oder EDMFA und HCN hergestellt werden. Die Aufarbeitung erfolgt, vorzusweise durch i) Abtrennung von Leichtsiedern, beispielsweise durch Strippen, Entspannungsverdampfung oder Destillation und ii) Abreicherung von Wasser, vorzugsweise in Gegenwart eines organischem Lösungsmittels, welches unter den Bedingungen der Wasserabtrennung einen Siedepunkt zwischen Wasser und EDDN bzw. EDMN aufweist oder welches ein mit Wasser leicht siedendes Azeotrop bildet.

Das entwässerte EDDN wird vorzugsweise mit THF vermischt und mit Adsorbens, vorzugsweise festem, sauren Adsorbens behandelt. Das Gemisch (1) von EDDN bzw. EDMN und THF wird in einem Hydrierreaktor (I) in Gegenwart von zugeführtem Wasserstoff (2) zu TETA bzw.

DETA hydriert. Der Reaktionsaustrag aus der Hydrierung (3) wird in einen Entspannungsbehälter (II) entspannt. Die gasförmigen Bestandteile (4), wie Wasserstoff, Teile des THF, HCN, Methanol oder Methylamin, können aus dem Verfahren ausgeschleust werden oder teilweise oder ganz zurück gewonnen werden.

Die nach der Entspannung flüssig verbliebene Phase (5) wird in eine Kolonne K1 geleitet, die einen Abtriebs- und einen Verstärkungsteil aufweist. Am Kopf der Kolonne wird ein leichtsiedendes THF/Wasser-Azeotrop (6) abgezogen, und kondensiert. Der kondensierte Strom wird mit Toluol (7) in einem Phasentrenn-Gefäß vermischt. In dem Phasentrenngefäß bildet sich eine wässrige Phase (8) und eine THF/Toluol-Phase (9), die in die Kolonne K1 zurückgeführt wird.

Aus dem Sumpf der Kolonne K1 wird ein Strom (10) abgezogen, der TETA, DETA, THF, Toluol sowie organische Verbindungen, wie PIP, AEPIP und TEPA enthält.

Dieser Strom (10) wird in eine Kolonne K2 geleitet, in der THF als Kopfprodukt (11) abgezogen wird. Dieses THF (11) kann direkt in das Verfahren zurückgeführt werden, vorzugsweise in die Behandlung von EDDN bzw. EDMN mit Adsorbens. Vor der Einleitung in die Adsorber-Stufe kann das THF (11) mit einem Molsieb zur weiteren Abreicherung von Wasser in Kontakt gebracht werden.

Am Sumpf der Kolonne K2 wird ein Strom (12) abgezogen, der TETA, DETA, Toluol sowie organische Verbindungen, wie PIP, AEPIP und TEPA enthält.

Dieser Strom (12) wird in eine Kolonne K3 eingeleitet, in der Toluol am Kopf (13) abgezogen wird. Das abgezogene Toluol (13) kann zur Entwässerung von THF über Leitung (7) in ein Phasentrenn-Gefäß geleitet werden, in dem es mit dem Kondensat (6) aus Kolonne K1 vereinigt wird. Das abgezogene Toluol (13) kann auch über Leitung (14) aus dem Verfahren ausgeschleust werden oder vorzugsweise als Lösungsmittel bei der EDDN und/oder EDMN-Herstellung eingesetzt werden.

Das Sumpfprodukt der Kolonne K3 (16) enthält TETA, DETA, Toluol sowie organische Verbindungen, wie PIP, AEPIP und TEPA. Dieses Gemisch kann in der Kolonne K4 weiter aufgetrennt werden. Beispielsweise können Leichtsieder, wie PIP, AEPIP und DETA über Kopf (17) abgezogen werden und TETA als Seitenabzug (18) entnommen werden. Hochsieder, wie TEPA, können am Sumpf (19) abgezogen werden. Der Kopf- bzw. der Sumpfstrom kann in nachfolgenden Destillationsstufen in seine Einzelbestandteile aufgetrennt werden.

Abkürzungen

Ethylendiamin (EDA)
Ethylendiamin-Formaldehyd-Bisaddukt (EDFA)
Ethylendiamin-Formaldehyd-Monoaddukt (EDMFA)
Ethylendiamindiacetonitril (EDDN)
Ethylendiaminmonoacetonitril (EDMN)
Diethylentriamin (DETA)
Triethylentetramin (TETA)
Tetraethylenpentamin (TEPA)
Formaldehyd (FA)
Formaldehydcyanhydrin (FACH)
Piperazin (PIP)
Aminoethylpiperazin (AEPIP)
Gemisch aus Formaldehyd und Blausäure (GFB)

2- und 3- Methyltetrahydrofuran (MeTHF)

Aminoacetonitril (AAN)

Das erfindungsgemäße Verfahren wird anhand der nachfolgend aufgeführten Beispiele näher ausgeführt.

Beispiele

Analytik

Der Formaldehydcyanhydrin (FACH)- und der Blausäure-Umsatz wurden durch Volhard-Titration (Bestimmung von freiem Cyanid) und Liebig-Titration (Bestimmung von gebundenem Cyanid) ermittelt. Bei beiden Methoden wurde mit Silbernitrat titriert. Die Ausbeute an Wertprodukten wurde per quantitativer HPLC-Analyse (feste Phase: 3 x Atlantis T3, 5 $\mu$, 4.6 x 250 mm, Waters; mobile Phase: 50 Vol.-% Wasser mit 0.5 g/L Ammoniumformiat, 50 Vol.-% Acetonitril) mit den jeweils als Reinstoff vorliegenden Reaktionsprodukten bzw. Vergleichssubstanzen ermittelt. Als Wertprodukt wird die Summe der $\alpha$-Aminonitrile Ethylendiamindiacetonitril (EDDN), Ethylendiaminmonoacetonitril (EDMN), Biscyanomethylimidazolin (BCMI) und Ethylendiamintriacetonitril (EDTriN) angegeben. Bei der Bestimmung der Hazen (APHA)- und lod-Farbzahl (Römpp, Lexikon Chemie, 10. Auflage, G. Thieme Verlag, 1997, Seiten 1285 bis 1286) wurde jeweils der wässrige Teil des Reaktionsaustrags ggf. nach Phasentrennung vermessen.

Allgemeine Herstellvorschriften

Schiff Base (EDFA)- Synthese

700 g/h Formaldehyd (Strom 1, 30 Gew.-% in Wasser) und 209 g/h Ethylendiamin (Strom 2, rein) wurden durch einen Schlaufenreaktor I mit 138 mL Volumen geleitet. Die umgepumpte Menge im Schlaufenreaktor betrug 15 kg/h, die Temperatur wurde auf 45 °C eingestellt und der Druck lag bei 1.6 bar. Ein Teil des Schlaufenreaktorinhalts (Strom 3) wurde kontinuierlich ausgeschleust und durch den nachgeschalteten Rohrreaktor II mit 19 mL Volumen geleitet.

Der Reaktoraustrag wurde nicht analysiert und unmittelbar weiter umgesetzt (Beispiel 1).

Beispiel 1

Die gemäß der allgemeinen Herstellvorschrift hergestellte Schiff Base (Strom 4, 909 g/h, ca. 46 Gew.-% in Wasser, temperiert auf 45 °C, Dichte ca. 900 g/L) wurde mit 205 g/h HCN (Strom 5, 90 Gew.-% in Wasser) und 1.1 kg/h Toluol bzw. organischer Phase (Strom 6) durch den Rohrreaktor III mit 16 mL Volumen geführt. Am Ausgang des Rohrreaktors betrug die Temperatur 75 °C und der Druck 1.5 bar. Die Verweilzeit im Rohrreaktor III betrug 23 Sekunden (die Bezeichnungen der Apparate und Ströme erfolgt analog Figur 1).

Der Reaktoraustrag wurde nicht analysiert und unmittelbar nach der Umsetzung wie folgt aufgearbeitet:

> Der Austrag aus dem Rohrreaktor III (Strom 7) wurde in die Flashvorlage IV auf 0.15 bar entspannt. Dabei kühlte er sich auf 43 °C ab. Die Brüden (Strom 8) wurden auf den Kondensator V und der flüssige Teil (Strom 12) auf den Kopf der Destillationskolonne VII abgeführt. Die Kolonne VII enthielt 960 mm Packung Montz A3-500 mit einem Durchmesser von 42 mm. Die Höhe der Packung entspricht einer theoretischen Trennstufenzahl von ca. 4. Am Kolonnenkopf wurde eine Temperatur von 41 °C gemessen. Die Brüden (Strom 13) der Kolonne VII wurden ebenfalls am Kondensator V kondensiert. Das Kondensat (Strom 10) zerfiel dabei in eine wässrige (Strom 14) und eine organische Phase (Strom 11), die im Abscheider VI getrennt wurden. Die Menge an wässriger Phase (Strom 14) betrug 635 g/h. Diese wurde komplett ausgeschleust. Von der organischen Phase wurden insgesamt 3.9 kg/h (Strom 11) zurückgeführt. 1.1 kg/h davon (Strom 21) wurden wieder zur Kühlung des Rohrreaktors III genutzt, der Rest auf den Kopf der Kolonne VII zur Wasserabtrennung zurückgeführt. Toluolverluste wurden durch Zusatz von reinem Toluol ausgeglichen. Am Sumpf der Kolonne VII wurde eine Temperatur von 69 °C eingestellt und eine Menge von 542 g/h (Strom 17) abgezogen.

Der Gew.-% EDMN, 70.50 Gew.-% EDDN, 7.65 Gew.-% BCMI, 0.56 Gew.-% EDTriN und 0.11 Gew.-% Wasser. Als lodfarbzahl wurde 38.1 bestimmt.

Bezüglich eingesetzten Ethylendiamins wurden über die gesamten oben aufgeführten Schritte die folgenden Ausbeuten erhalten: 11.2% EDMN, 79.5% EDDN, 7.9% BCMI, 0.5% EDTriN, 99.1 % Gesamtausbeute an Wertprodukten. Der Sumpfaustrag (Strom 17) hatte die folgende Zusammensetzung: 10.56 Gew.-% Toluol, 7.13 Gew.-% EDMN, 70.50 Gew.-% EDDN, 7.65 Gew.-% BCMI, 0.56 Gew.-% EDTriN und 0.11 Gew.-% Wasser. Als lodfarbzahl wurde 38.1 bestimmt.

Beispiel 2 (Vergleichsbeispiel)

Analog Beispiel 2 der WO 2008/104582 wurden in einem 2 L Reaktionsgefäß Ethylendiamin (132 g, 2.2 mol) vorgelegt und unter Eiskühlung bei einer Temperatur von maximal 30 °C innerhalb von 2 h Formaldehydcyanhydrin (563 g, 4.29 mol) zugetropft. Nach 4.5 h Nachrührzeit wurde die Lösung per Volhard- und Liebig-Titrationen, HPLC sowie Farbzahlmessung analysiert. Der Formaldehydcyanhydrinumsatz betrug laut Volhard- und Liebig-Titrationen 99.8%. Als Gesamtaminonitrilausbeute wurde per HPLC 97.6%, bezogen auf eingesetztes Formaldehydcyanhydrin, ermittelt. Die Farbzahl der wässrigen Lösung betrug 10.1 Iod. Die Hazen-Farbzahl war nicht mehr bestimmbar (Tabelle 1).

Tabelle 1:

| Titrationen [Gew.-%] | | HPLC [Gew.-%] | | | | Ausbeute bez. EDA [%] | | | | | FZ | |
|---|---|---|---|---|---|---|---|---|---|---|---|---|
| Volhard | Liebig | EDMN | EDDN | BCMI | EDTriN | EDMN | EDDN | BCMI | EDTriN | gesamt | [Iod] | [Hazen] |
| 0.01 | 0.05 | 1.8 | 38.6 | 1.9 | 0.0 | 5.9 | 88.2 | 4.1 | 0.0 | 98.2 | 10.1 | n.bb. |
| | | | | | | Ausbeute bez. FACH [%] | | | | | | |
| | | | | | | 3.0 | 90.4 | 4.2 | 0.0 | 97.6 | | |

Die oben aufgeführten Beispiele zeigen, dass das erfindungsgemäße Verfahren ermöglicht die Umsetzung von EDFA mit HCN mit hohen Umsätzen und Ausbeuten durchzuführen. Es wird ein Reaktionsaustrag mit geringen Verfärbungen und Nebenprodukten erhalten. Die kurze Verweilzeit im Rohrreaktor ermöglicht die Reaktion in einem relativ kleinen Reaktor durchzuführen, dessen Temperatur-Überwachung einfach ist und in dem der Anstieg der Reaktionstemperatur kontrolliert werden kann.

Beispiel 3: Stabilität von 40 Gew.-% EDDN in Wasser bei 40, 60, 80 und 100 °C bis 600 s

In einem 250 mL Rundkolben mit Thermometer, Rückflusskühler und KPG Rührer wurde EDDN (30 g, rein) in Wasser (45 g) gelöst. Mit einem vorgeheizten Ölbad wurde die Lösung auf die in Tabelle 2 angegebene Temperatur erwärmt. Proben wurden zum Zeitpunkt t = 0, 300 und 600 s entnommen. Von jeder Probe wurde der EDDN Gehalt per HPLC sowie die Farbe des Gemischs per Farbzahlmessung bestimmt.

Tabelle 2:

| T [°C] | EDDN [Gew.-%] | | | EDDN [normierte Gew.-%] | | |
|---|---|---|---|---|---|---|
| | t = 0 s | t = 300 s | t = 600 s | t = 0 s | t = 300 s | t = 600 s |
| 40 | 40.3 | 39.1 | 39.6 | 100.0 | 97.1 | 98.2 |
| 60 | 39.8 | 40.3 | 39.9 | 100.0 | 101.4 | 100.4 |
| 80 | 39.5 | 38.4 | 38.1 | 100.0 | 97.1 | 96.4 |
| 100 | 39.7 | 30.2 | 25.3 | 100.0 | 76.1 | 63.9 |

| T [°C] | Farbzahl [Iod] | | | Farbzahl [Gardner] | | |
|---|---|---|---|---|---|---|
| | t = 0 s | t = 300 s | t = 600 s | t = 0 s | t = 300 s | t = 600 s |
| 40 | 6.5 | 6.9 | 7.0 | 5.3 | 5.4 | 5.5 |
| 60 | 6.4 | 7.8 | 8.3 | 5.2 | 5.7 | 5.9 |
| 80 | 6.4 | 10.9 | 17.3 | 5.2 | 6.5 | 7.8 |
| 100 | 6.4 | 43.1 | 77.4 | 5.2 | 9.1 | 10.6 |

Beispiel 4: Stabilität von 40 Gew.-% EDDN in Wasser bei 40, 60, 80 und 100 °C bis 120 min

In einem 250 mL Rundkolben mit Thermometer, Rückflusskühler und KPG Rührer wurde EDDN (30 g, rein) in Wasser (45 g) gelöst. Mit einem vorgeheizten Ölbad wurde die Lösung auf die in den Tabellen 3 angegebene Temperatur erwärmt. Proben wurden zum Zeitpunkt t = 0, 5, 10, 30, 60, 90 und 120 min entnommen. Von jeder Probe wurde der EDDN Gehalt per HPLC sowie die Farbe des Gemischs per Farbzahlmessung bestimmt.

Tabellen 3

| 40 °C | EDDN | | Farbzahl | |
|---|---|---|---|---|
| t [min] | [Gew.-%] | [normierte Gew.-%] | [Iod] | [Gardner] |
| 0 | 40.3 | 100.0 | 6.5 | 5.3 |
| 5 | 39.1 | 97.1 | 6.9 | 5.4 |
| 10 | 39.6 | 98.2 | 7.0 | 5.5 |
| 30 | 39.3 | 97.6 | 7.2 | 5.5 |
| 60 | 39.8 | 98.9 | 7.5 | 5.7 |

(fortgesetzt)

| 40 °C | EDDN | | Farbzahl | |
|---|---|---|---|---|
| t [min] | [Gew.-%] | [normierte Gew.-%] | [Iod] | [Gardner] |
| 90 | 40.8 | 101.3 | 7.8 | 5.8 |
| 120 | 39.4 | 97.8 | 8.1 | 5.9 |
| 60 °C | EDDN | | Farbzahl | |
| t [min] | [Gew.-%] | [normierte Gew.-%] | [Iod] | [Gardner] |
| 0 | 39.8 | 100.0 | 6.4 | 5.2 |
| 5 | 40.3 | 101.4 | 7.8 | 5.7 |
| 10 | 39.9 | 100.4 | 8.3 | 5.9 |
| 30 | 37.6 | 94.5 | 12.1 | 6.7 |
| 60 | 37.3 | 93.7 | 79.7 | 10.7 |
| 90 | 36.3 | 91.4 | n.b. | 14.3 |
| 120 | 35.6 | 89.9 | n.b. | 17.0 |
| 80 °C | EDDN | | Farbzahl | |
| t [min] | [Gew.-%] | [normierte Gew.-%] | [Iod] | [Gardner] |
| 0 | 39.5 | 100.0 | 6.4 | 5.2 |
| 5 | 38.4 | 97.1 | 10.9 | 6.5 |
| 10 | 38.1 | 96.4 | 17.3 | 7.8 |
| 30 | 32.6 | 82.6 | n.b. | 11.7 |
| 60 | 28.5 | 72.2 | n.b. | 16.9 |
| 90 | 22.8 | 57.8 | n.b. | n.b. |
| 120 | 18.7 | 47.3 | n.b. | n.b. |
| 100 °C | EDDN | | Farbzahl | |
| t [min] | [Gew.-%] | [normierte Gew.-%] | [Iod] | [Gardner] |
| 0 | 39.7 | 100.0 | 6.4 | 5.2 |
| 5 | 30.2 | 76.1 | 43.1 | 9.1 |
| 10 | 25.3 | 63.9 | 77.4 | 10.6 |
| 30 | 18.0 | 45.4 | n.b. | 15.3 |
| 60 | 10.6 | 26.6 | n.b. | 17.5 |
| 90 | 6.1 | 15.3 | n.b. | n.b. |
| 120 | 3.8 | 9.5 | n.b. | n.b. |

Beispiel 5: Stabilität von EDDN in 5, 20 und 40 Gew.-% Wasser bei 80 °C bis 120 min

In einem 250 mL Rundkolben mit Thermometer, Rückflusskühler und KPG Rührer wurde EDDN (rein) mit der angegebenen Wassermenge versetzt. Mit einem vorgeheizten Ölbad wurde die Lösung auf 80 °C erwärmt. Proben wurden zum Zeitpunkt t = 0 und 120 min entnommen. Von jeder Probe wurde die Farbe des Gemischs per Farbzahlmessung bestimmt (Tabelle 4).

Tabelle 4:

| EDDN [g] | Wasser | | Farbzahl [Gardner] | |
|---|---|---|---|---|
| | [g] | [Gew.-%] | t = 0 min | t=120min |
| 80 | - | 0 | 6.0 | 8.6 |
| 76 | 4 | 5 | 5.8 | 14.8 |
| 60 | 15 | 20 | 5.2 | n.b. |
| 30 | 45 | 40 | 5.2 | n.b. |

**Patentansprüche**

1. Verfahren zur Umsetzung von Ethylendiamin-Formaldehyd-Addukt (EDFA) und/oder Ethylendiamin-Monoformaldehyd-Addukt (EDMFA) mit Blausäure (HCN) in einem Reaktor mit begrenzter Rückvermischung bei einer Temperatur im Bereich von 20 bis 120°C, **dadurch gekennzeichnet, dass** die Verweilzeit im Reaktor 300 Sekunden oder weniger beträgt.

2. Verfahren nach Anspruch 1, **dadurch gekennzeichnet, dass** die Verweilzeit im Reaktor weniger als 60 Sekunden beträgt.

3. Verfahren nach mindestens einem der Ansprüche 1 bis 2, **dadurch gekennzeichnet, dass** der Reaktor ein Rohrreaktor ist.

4. Verfahren nach mindestens einem der Ansprüche 1 bis 3, **dadurch gekennzeichnet, dass** die Eintrittstemperatur der Edukte in den Reaktor im Bereich von 10 bis 50°C liegt.

5. Verfahren nach mindestens einem der Ansprüche 1 bis 4, **dadurch gekennzeichnet, dass** die Reaktion in Gegenwart von einem oder mehreren organischen Lösungsmitteln erfolgt.

6. Verfahren nach mindestens einem der Ansprüche 1 bis 5, **dadurch gekennzeichnet, dass** die Umsetzung in Gegenwart von Wasser durchgeführt wird.

7. Verfahren nach mindestens einem der Ansprüche 1 bis 6, **dadurch gekennzeichnet, dass** man das Reaktionsgemisch nach Austritt aus dem Reaktor abkühlt.

8. Verfahren nach mindestens einem der Ansprüche 1 bis 7, **dadurch gekennzeichnet, dass** die Abkühlung des Reaktionsgemischs durch Entspannungsverdampfung erfolgt.

9. Verfahren nach Anspruch 8, **dadurch gekennzeichnet, dass** der Druck im Entspannungsverdampfungsschritt weniger als 300 mbar beträgt.

10. Verfahren nach mindestens einem der Ansprüche 5 bis 9, **dadurch gekennzeichnet, dass** das organische Lösungsmittel eine Mischungslücke mit Wasser aufweist.

11. Verfahren nach Anspruch 10, **dadurch gekennzeichnet, dass** das organische Lösungsmittel, welches eine Mischungslücke mit Wasser aufweist, Toluol ist.

12. Verfahren nach mindestens einem der Ansprüche 1 bis 11, **dadurch gekennzeichnet, dass** der Rohrreaktor zusätzlich über einen Kühlmantel gekühlt wird.

13. Verfahren nach Anspruch 8 und einem der Ansprüche 11 oder 12, **dadurch gekennzeichnet, dass** die nach der Entspannungsverdampfung gasförmigen Komponenten kondensiert werden und in einem Phasenscheider in eine wässrige und eine organische Phase getrennt werden, wobei die organische Phase zusammen mit den bei der Entspannungsverdampfung flüssig gebliebenen Komponenten einer Destillationsvorrichtung zugeführt werden, in der EDDN bzw. EDMN als Sumpfprodukt abgezogen werden.

## Claims

1. A process for reacting ethylenediamine-formaldehyde adduct (EDFA) and/or ethylenediamine-monoformaldehyde adduct (EDMFA) with hydrogen cyanide (HCN) in a reactor with limited backmixing at a temperature in the range from 20 to 120°C, wherein the residence time in the reactor is 300 seconds or less.

2. The process according to claim 1, wherein the residence time in the reactor is less than 60 seconds.

3. The process according to at least one of claims 1 and 2, wherein the reactor is a tubular reactor.

4. The process according to at least one of claims 1 to 3, wherein the inlet temperature of the reactants into the reactor is in the range from 10 to 50°C.

5. The process according to at least one of claims 1 to 4, wherein the reaction is effected in the presence of one or more organic solvents.

6. The process according to at least one of claims 1 to 5, wherein the reaction is performed in the presence of water.

7. The process according to at least one of claims 1 to 6, wherein the reaction mixture is cooled after leaving the reactor.

8. The process according to at least one of claims 1 to 7, wherein the reaction mixture is cooled by flash evaporation.

9. The process according to claim 8, wherein the pressure in the flash evaporation step is less than 300 mbar.

10. The process according to at least one of claims 5 to 9, wherein the organic solvent has a miscibility gap with water.

11. The process according to claim 10, wherein the organic solvent which has a miscibility gap with water is toluene.

12. The process according to at least one of claims 1 to 11, wherein the tubular reactor is additionally cooled by means of a cooling jacket.

13. The process according to claim 8 and either of claims 11 and 12, wherein the components that are gaseous after the flash evaporation are condensed and separated, in a phase separator, into an aqueous phase and an organic phase, the organic phase being supplied, together with the components that have remained liquid in the flash evaporation, to a distillation means, in which EDDN and/or EDMN are taken off as bottom product.

## Revendications

1. Procédé pour la transformation d'un produit d'addition d'éthylènediamine-formaldéhyde (EDFA) et/ou d'un produit d'addition d'éthylènediamine-monoformaldéhyde (EDMFA) avec de l'acide cyanhydrique (HCN) dans un réacteur avec un mélange en retour limité à une température dans la plage de 20 à 120°C, **caractérisé en ce que** le temps de séjour dans le réacteur est de 300 secondes ou moins.

2. Procédé selon la revendication 1, **caractérisé en ce que** le temps de séjour dans le réacteur est inférieur à 60 secondes.

3. Procédé selon au moins l'une quelconque des revendications 1 à 2, **caractérisé en ce que** le réacteur est un réacteur tubulaire.

4. Procédé selon au moins l'une quelconque des revendications 1 à 3, **caractérisé en ce que** la température d'entrée des produits de départ dans le réacteur se situe dans la plage de 10 à 50°C.

5. Procédé selon au moins l'une quelconque des revendications 1 à 4, **caractérisé en ce que** la réaction a lieu en présence d'un ou de plusieurs solvants organiques.

6. Procédé selon au moins l'une quelconque des revendications 1 à 5, **caractérisé en ce que** la transformation est réalisée en présence d'eau.

**7.** Procédé selon au moins l'une quelconque des revendications 1 à 6, **caractérisé en ce qu'**on refroidit le mélange réactionnel après la sortie du réacteur.

**8.** Procédé selon au moins l'une quelconque des revendications 1 à 7, **caractérisé en ce que** le refroidissement du mélange réactionnel a lieu par une évaporation par détente.

**9.** Procédé selon la revendication 8, **caractérisé en ce que** la pression dans l'étape d'évaporation par détente est inférieure 300 mbars.

**10.** Procédé selon au moins l'une quelconque des revendications 5 à 9, **caractérisé en ce que** le solvant organique présente une lacune de mélange avec l'eau.

**11.** Procédé selon la revendication 10, **caractérisé en ce que** le solvant organique, qui présente une lacune de mélange avec l'eau, est le toluène.

**12.** Procédé selon au moins l'une quelconque des revendications 1 à 11, **caractérisé en ce que** le réacteur tubulaire est en outre refroidi via une enveloppe de refroidissement.

**13.** Procédé selon la revendication 8 et selon l'une quelconque des revendications 11 ou 12, **caractérisé en ce que** les composants gazeux après l'évaporation par détente sont condensés et séparés dans un séparateur de phases en une phase aqueuse et une phase organique, la phase organique étant introduite ensemble avec les composants qui sont restés liquides lors de l'évaporation par détente dans un dispositif de distillation dans lequel l'EDDN ou l'EDMN sont soutirés comme produit de fond.

Figur 1

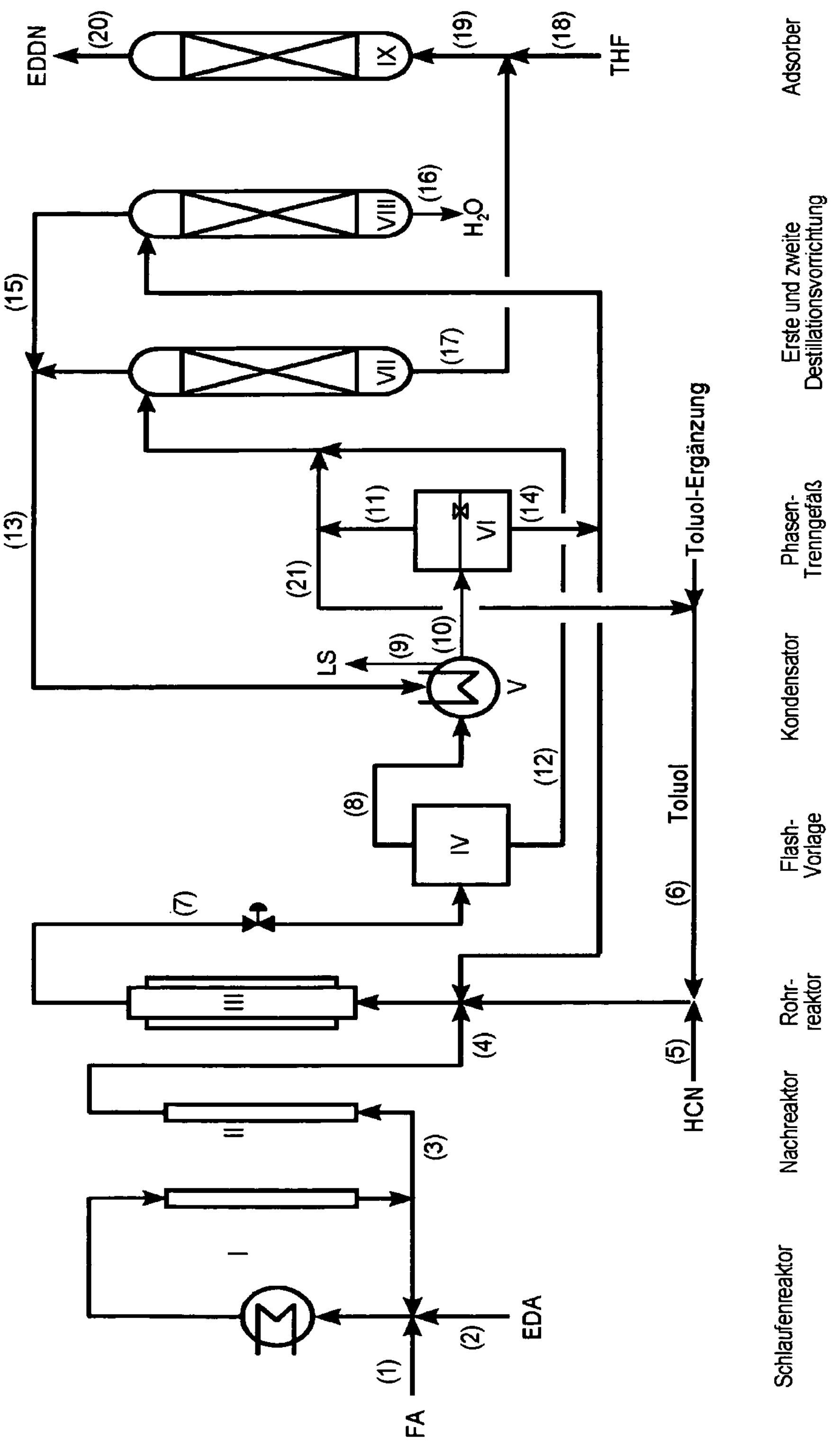
EDDN
(20)
IX
(19)
(18)
THF
Adsorber
VIII
(16)
$H_2O$
(15)
VII
(17)
Erste und zweite Destillationsvorrichtung
(13)
(11)
VI
(14)
(21)
Toluol-Ergänzung
Phasen-Trenngefäß
LS
(9)
(10)
V
Kondensator
(12)
(8)
IV
Toluol
Flash-Vorlage
(7)
(6)
III
Rohrreaktor
(4)
(5)
HCN
II
(3)
Nachreaktor
I
(1)
(2)
FA
EDA
Schlaufenreaktor

Figur 2

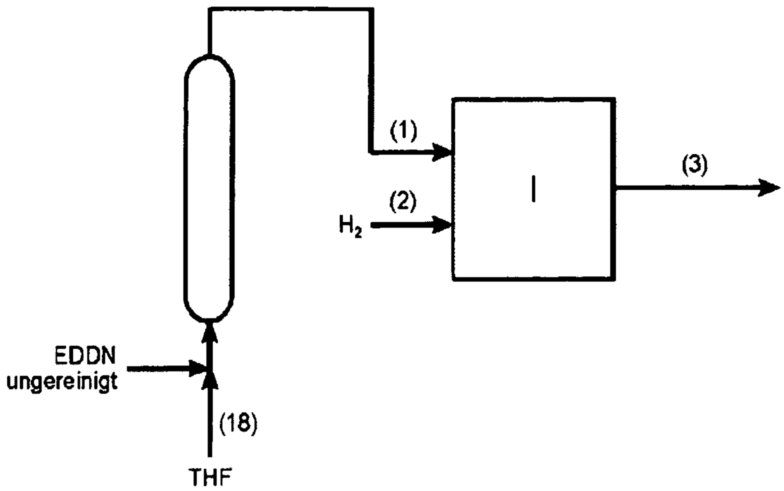


EDDN-Reinigung EDDN-Hydrierung

Figur 3

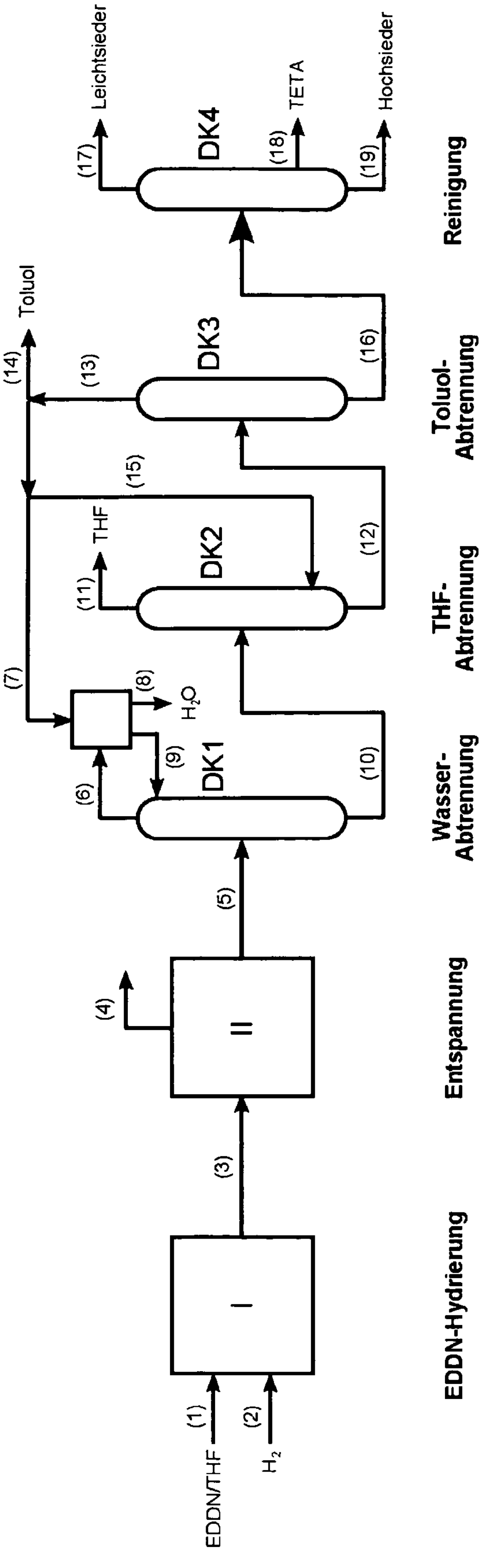
EDDN/THF
(1)
H2
(2)
I
EDDN-Hydrierung
(3)
II
(4)
Entspannung
(5)
DK1
(6)
(7)
(8)
H2O
(9)
(10)
Wasser-Abtrennung
DK2
(11)
THF
(12)
(15)
THF-Abtrennung
DK3
(13)
(14)
Toluol
(16)
Toluol-Abtrennung
DK4
(17)
Leichtsieder
(18)
TETA
(19)
Hochsieder
Reinigung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 61529290 B
- WO 2008104579 A
- WO 2008104583 A
- EP 1209146 A
- EP 1742045 A
- EP 963975 A
- EP 696572 A
- WO 9944984 A
- WO 9933561 A
- EP 892777 A
- WO 2008104553 A
- WO 2008104582 A


### In der Beschreibung aufgeführte Nicht-Patentliteratur


- Amines, aliphatic. **KARSTEN ELLER ; ERHARD HENKES ; ROLAND ROSSBACHER ; HARTMUT HÖKE.** Ullmann's Encyclopedia of Industrial Chemistry. 15. Juni 2000, 33
- **HANS-JÜRGEN ARPE.** Industrielle Organische Chemie. Wiley VCH, 2007
- **RÖMPP.** Lexikon Chemie. G. Thieme Verlag, 1997, 1285-1286